# EUROPEAN PATENT SPECIFICATION

(11) **EP 1 680 387 B1**
(45) Date of publication and mention of the grant of the patent: **12.09.2007**
(21) Application number: 04795267.6
(22) Date of filing: 14.10.2004
(51) Int. Cl.: C07C 29/16, C07C 31/125

(54) **PREPARATION OF BRANCHED ALIPHATIC ALCOHOLS USING A PROCESS STREAM FROM A DEHYDROGENATION-ISOMERIZATION UNIT**
HERSTELLUNG VON VERZWEIGTEN ALIPHATISCHEN ALKOHOLEN UNTER VERWENDUNG EINES VERFAHRENSSTROMS AUS EINER DEHYDRIERUNGS-ISOMERISIERUNGS-ANLAGE
PREPARATION D'ALCOOLS ALIPHATIQUES RAMIFIES AU MOYEN D'UN COURANT DE PROCEDE PROVENANT D'UNE UNITE D'ISOMERISATION-DESHYDROGENATION

(30) Priority: 15.10.2003 US 511524 P
(43) Date of publication of application: 19.07.2006
(73) Proprietor: Shell Internationale Research Maatschappij B.V., 2596 HR The Hague (NL)
(72) Inventor: AYOUB, Paul, Marie, NL-1031 CM Amsterdam (NL); DIRKZWAGER, Hendrik, NL-1031 CM Amsterdam (NL); MURRAY, Brendan, Dermot, Houston, TX 77077 (US); SUMROW, Steven, Clois, Katy, TX 77450 (US)
(86) International application number: PCT/US2004/034080
(87) International publication number: WO 2005/037752

(56) References cited:
- GB-A- 1 601 818
- US-A- 3 907 909
- US-A- 4 438 287

## Description

The present invention generally relates to systems and methods for preparing aliphatic alcohols. More particularly, embodiments described herein relate to systems and methods for preparing branched aliphatic alcohols using a dehydrogenation-isomerization unit.

### Description of Related Art

Aliphatic alcohols are important compounds that may be used in a variety of applications or converted to other chemical compounds (e.g., surfactants, sulfates). Surfactants may be used in a variety of applications (e.g., detergents, soaps, oil recovery).

The structural composition of the aliphatic alcohol may influence the properties of the surfactant and/or detergent (e.g., water solubility, biodegradability and cold water detergency) produced from the aliphatic alcohol. For example, water solubility may be affected by the linearity of the aliphatic portion of the aliphatic alcohol. As the linearity of the aliphatic portion increases, the hydrophilicity (i.e., affinity for water) of the aliphatic alcohol surfactant may decrease. Thus, the water solubility and/or detergency performance of the aliphatic alcohol surfactant may decrease. Incorporating branches into the aliphatic portion of the aliphatic alcohol surfactant may increase the cold-water solubility and/or detergency of the aliphatic alcohol surfactant. Biodegradability, however, of the aliphatic alcohol surfactants may be reduced if the branches in the aliphatic portion of the alcohol surfactant include a high number of quaternary carbons. Incorporation of branches with a minimum number of quaternary carbon atoms into the aliphatic portion of the aliphatic alcohol surfactant may increase cold-water solubility and/or detergency of the alcohol surfactants while maintaining the biodegradability properties of the detergents.

The aliphatic portion of an aliphatic alcohol used to manufacture a surfactant may include one or more aliphatic alkyl groups as branches. Aliphatic alkyl groups that may form branches in the aliphatic portion may include methyl, ethyl, propyl or higher alkyl groups. Quaternary and tertiary carbons may be present when the aliphatic portion is branched. The number of quaternary and tertiary carbons may result from the branching pattern in the aliphatic portion. As used herein, the phrase "aliphatic quaternary carbon atom" refers to a carbon atom that is not bound to any hydrogen atoms.

Processes to manufacture branched primary alcohol compositions are described by U.S. Patent No. 5,849,960 to Singleton et al. entitled "Highly Branched Primary Alcohol Compositions, and Biodegradable Detergents Made Therefrom" and U.S Patent No. 6,150,322 to Singleton et al., entitled "Highly Branched Primary Alcohol Compositions, and Biodegradable Detergents Made Therefrom." US 4,438,287 describes a process for synthesising alcohols in which a dehydrogenatable hydrocarbon is subjected to a dehydrogenation step in the presence of a nonacidic multimetallic catalyst. The product mixture is then utilised, without a separation of the olefins resulting from the hydrogenation process, as a feedstock for an alcohol synthesis step.

### Summary of the Invention

In an embodiment, aliphatic alcohols may be produced by a method that includes dehydrogenation of paraffins to olefins and isomerization of the olefins in a dehydrogenation-isomerization unit. A process feed stream entering a dehydrogenation-isomerization unit may include linear olefins and paraffins having an average carbon number from 7 to 18. In an embodiment, a process feed stream entering a dehydrogenation-isomerization unit includes linear olefins and paraffins having an average carbon number from 10 to 17. As used herein, the phrase "carbon number" refers to the total number of carbon atoms in a molecule. The process feed stream entering a dehydrogenation-isomerization unit, in some embodiments, is derived from a Fischer-Tropsch process.

At least a portion of the paraffins in the feed stream may be dehydrogenated to form olefins in the dehydrogenation-isomerization unit. At least a portion of the resulting olefins and at least a portion of the olefins that were already present in the feed stream may also be isomerized in the dehydrogenation-isomerization unit. An isomerization process converts linear olefins (e.g., unbranched olefins) into branched olefins. The isomerized olefins may be hydroformylated to produce aliphatic alcohols After hydroformylation of the olefins, unreacted components from the hydroformylation process may be separated from the aliphatic alcohol products. Paraffins and unreacted olefins in the separated stream may be recycled back into the dehydrogenation-isomerization unit.

Process conditions in the dehydrogenation-isomerization unit may be such that the resulting branched olefins have an average number of branches per olefin molecule from 0.7 to 2.5. The branched olefins may include, but are not limited to, methyl and/or ethyl branched olefins. The isomerization process may produce branched olefins that include less than 0.5 percent of quaternary aliphatic carbon atoms. The dehydrogenation isomerization unit may include a catalyst that has two functions, to dehydrogenate the paraffins to olefins and to isomerize the olefins into branched olefins.

In an embodiment, a dehydrogenation-isomerization unit may include a plurality of zones. The plurality of zones may include a first reaction zone and a second reaction zone. The first reaction zone may be a dehydrogenation zone. The second reaction zone may be an isomerization zone. A hydrocarbon stream, containing olefins and paraffins, may enter the dehydrogenation zone. At least a portion of the paraffins in the hydrocarbon stream may be dehydrogenated to olefins to produce a stream enriched in olefins. The enriched olefin stream may be passed into the isomerization zone. In the isomerization zone, at least a portion of the olefins in the enriched olefin stream may be isomerized to branched olefins. The branched olefins may be converted to aliphatic alcohols by hydroformylation. After hydroformylation of the olefins, a paraffins and unreacted olefins stream may be separated from the produced aliphatic alcohol products. The paraffins and unreacted olefins stream may be recycled by directing at least a portion of the paraffins and unreacted olefins stream back into the dehydrogenation-isomerization unit and/or into a stream entering the dehydrogenation-isomerization unit.

In an embodiment, a dehydrogenation-isomerization unit may include a stacked bed configuration. The stacked bed may include a catalyst for dehydrogenation and a catalyst for isomerization of a hydrocarbon stream. At least a portion of the paraffins in the hydrocarbon stream may be dehydrogenated to olefins to produce a stream enriched in olefins. The enriched olefin stream may be passed into an isomerization zone. In the isomerization zone, at least a portion of the olefins in the enriched olefin stream may be isomerized to branched olefins. The branched olefins may be converted to aliphatic alcohols by hydroformylation. After hydroformylation of the olefins, a paraffins and unreacted olefins stream may be separated from the produced aliphatic alcohol products. The paraffins and unreacted olefins stream may be recycled by directing at least a portion of the paraffins and unreacted olefins stream back into the dehydrogenation-isomerization unit and/or into a stream entering the dehydrogenation-isomerization unit.

In certain embodiments, at least a portion of the aliphatic alcohols may be sulfated to form aliphatic sulfates. In some embodiments, aliphatic sulfates may include branched alkyl groups.

In certain embodiments, at least a portion of the produced aliphatic alcohols may be oxyalkylated to form oxyalkyl alcohols. In some embodiments, oxyalkyl alcohols may include branched alkyl groups. In some embodiments, at least a portion of the produced branched aliphatic alcohols may be ethoxylated to form branched ethoxyalkyl alcohols. At least a portion of the oxyalkyl alcohols may be sulfated to from oxyalkyl sulfates. In some embodiments, oxyalkyl sulfates may include branched alkyl groups.

### Brief Description of the Drawings

Advantages of the present invention will become apparent to those skilled in the art with the benefit of the following detailed description of embodiments and upon reference to the accompanying drawings, in which:
FIG. 1 depicts a schematic diagram of an embodiment of a system for producing branched aliphatic alcohols using a dehydrogenation-isomerization unit.
FIG. 2 depicts a schematic diagram of an embodiment of a system for producing branched aliphatic alcohols using a dehydrogenation-isomerization unit and a separation unit to separate branched olefins from linear olefins and paraffins.
FIG. 3 depicts a schematic diagram of an embodiment of a system for producing branched aliphatic alcohols using a dehydrogenation-isomerization unit with addition of an additional hydrocarbon stream.
FIGS. 4 A-B depict schematic diagrams of embodiments of a system for producing branched aliphatic alcohols using a two-zone dehydrogenation-isomerization unit.
FIG. 5 depicts a schematic diagram of an embodiment of a system for producing branched aliphatic alcohols using a dehydrogenation-isomerization unit with a stacked bed catalyst configuration.

While the invention is susceptible to various modifications and alternative forms, specific embodiments thereof are shown by way of example in the drawing and will herein be described in detail. It should be understood that the drawings and detailed description thereto are not intended to limit the invention to the particular form disclosed, but on the contrary, the intention is to cover all modifications, equivalents, and alternatives falling within the spirit and scope of the present invention as defined by the appended claims.

### Detailed Description of Embodiments

Hydrocarbon products may be synthesized from synthesis gas (i.e., a mixture of hydrogen and carbon monoxide) using a Fischer-Tropsch process. Synthesis gas may be derived by partial combustion of petroleum (e.g., coal, hydrocarbons), by reforming of natural gas or by partial oxidation of natural gas. The Fischer-Tropsch process catalytically converts synthesis gas into a mixture of products that includes saturated hydrocarbons, unsaturated hydrocarbons and a minor amount of oxygen-containing products. The products from a Fischer-Tropsch process may be used for the production of fuels (e.g., gasoline, diesel oil), lubricating oils and waxes.

Fischer-Tropsch process streams may also be used to prepare commodity products, which have economic value. For example, linear olefins are commodity products that are useful for the production of surfactants. Using a portion of the process stream to produce linear olefins may increase the economic value of a Fischer-Tropsch process stream.

Surfactants derived from branched olefins may have different properties than surfactants derived from linear olefins. For example, surfactants derived from branched olefins may have increased water solubility and/or improved detergency properties compared to surfactants derived from linear olefins. Biodegradable properties of the surfactant, however, may be affected by the presence of quaternary carbon atoms in the branched portion of the surfactant. Surfactants made from branched olefins with a minimum number of quaternary carbon atoms may have similar biodegradable properties to surfactants derived from linear olefins. Production of branched olefins from a Fischer-Tropsch process stream may increase the economic value of the stream. In some embodiments, linear olefins may be converted into branched olefins with a minimum number of quaternary carbon atoms using a dehydrogenation-isomerization or an isomerization catalyst. Increasing the amount of branched olefins derived from a Fischer-Tropsch process stream may increase the economic value of the process streams.

Methods are described for increasing the amount of branched olefins derived from a process stream that includes certain amount of olefins, thus increasing the economic value of the process stream. Such methods are useful for both Fischer-Tropsch process streams and product streams from other sources that include hydrocarbons.

A hydrocarbon feed stream composition may include paraffins and olefins. At least a portion of the hydrocarbon stream may be made up of linear paraffins and olefins having at least 4 carbon atoms and up to 18 carbon atoms. A hydrocarbon feed stream may be obtained from a Fischer-Tropsch process or from an ethylene oligomerization process. Fischer-Tropsch catalysts and reaction conditions may be selected to provide a particular mix of products in the reaction product stream. For example, a Fischer-Tropsch catalyst and reaction conditions may be selected to increase the amount of olefins and decrease the amount of paraffins and oxygenates in the stream. Alternatively, the catalyst and reaction conditions may be selected to increase the amount of paraffins and decrease the amount of olefins and oxygenates in the stream.

The catalyst used in a Fischer-Tropsch process may be Mo, W, Group VIII compounds or combinations thereof. Group VIII compounds include, but are not limited to, iron, cobalt, ruthenium, rhodium, platinum, palladium, iridium and osmium. Catalysts and combinations for manufacture of hydrocarbon species by a Fischer-Tropsch process are generally known.

While reference is made to a Fischer-Tropsch stream, any stream of olefins and saturated hydrocarbons may be suitable. Many Fischer-Tropsch streams may contain from 5 percent to 80 percent olefins, the remainder being saturated hydrocarbons comprising paraffins and other compounds.

In some embodiments, feed streams containing olefins and paraffins are obtained through cracking of paraffin wax or the oligomerization of olefins. Commercial olefin products manufactured by ethylene oligomerization are marketed in the United States by Chevron Phillips Chemical Company, Shell Chemical Company (as NEODENE^{®}) and British Petroleum. Cracking of paraffin wax to produce alpha-olefin and paraffin feed streams is described in U.S. Patent No. 4,579,986 to Sie, entitled "Process For The Preparation Of Hydrocarbons" and U.S. Patent Application Serial No. 10/153,955 of Ansorge et al., entitled "Process For The Preparation of linear Olefins and Use Thereof To Prepare Linear Alcohols." Specific procedures for preparing linear olefins from ethylene are described in U.S. Patent No. 3,676,523 to Mason, entitled "Alpha-Olefin Production;" U.S. Patent No. 3,686,351 to Mason, entitled "Alpha-Olefin Production;" U.S. Patent No. 3,737,475 to Mason, entitled "Alpha-Olefin Production" and U.S. Patent No. 4,020,121 to Kister et al., entitled "Oligomerization Reaction System." Most of the above-mentioned processes produce alpha-olefins. Higher linear internal olefins may be commercially produced (e.g., chlorination-dehydrochlorination of paraffins, paraffin dehydrogenation, isomerization of alpha-olefins).

In an embodiment, a feed stream is processed to produce a hydrocarbon stream that includes branched olefins. These branched olefins may be converted to branched aliphatic alcohols using various techniques. The feed steam may have a paraffin content range between 50 percent by weight to 90 percent by weight of the feed stream. In certain embodiments, a feed stream may have a paraffin content greater than 90 percent by weight paraffins. The feed stream may also include olefins. The olefin content of the feed stream may be between 10 percent by weight to 50 percent by weight.

The composition of the feed stream may include hydrocarbons having an average carbon number ranging from 4 to 30. In an embodiment, an average carbon number of the hydrocarbons in a feed stream may range from 4 to 24. In other embodiments, an average carbon number of the feed stream may range from 4 to 18. An average carbon number of the hydrocarbons in a feed stream may range from 7 to 18. In certain embodiments, an average carbon number of the hydrocarbons in a feed stream may range from 10 to 17. In some embodiments, an average carbon number of hydrocarbons in a feed stream may range from 10 to 13. In other embodiments, an average carbon number of hydrocarbons in a feed stream may range from 14 to 17. A feed stream may include minor amounts of hydrocarbons having a carbon number that is higher or lower than the desired carbon number range. In some embodiments, a feed stream may be derived from distillation of a process stream that includes a broader range of carbon numbers.

In an embodiment, a feed stream for a dehydrogenation-isomerization unit includes mono-olefins and/or paraffins. The mono-olefins may be of a linear or branched structure. The mono-olefins may have an alpha or internal double bond position. The feed stream may include olefins in which 50 percent or more of the olefin molecules present may be alpha-olefins of a linear (straight chain) carbon skeletal structure. In certain embodiments, at least 70 percent of the olefins are alpha-olefins of a linear carbon skeletal structure. A hydrocarbon stream in which greater than 70 percent of all of the olefin molecules are alpha-olefins of a linear carbon skeletal structure may be used in certain embodiments to convert olefins to aliphatic alcohols. Such a stream may be derived from a Fischer-Tropsch process. In some embodiments, a feed stream includes olefins in which at least 50 percent of the olefin molecules present are internal olefins.

Branched chain olefins may be converted to branched aliphatic alcohols (e.g., branched primary alcohols) by a hydroformylation process. "Hydroformylation," as used herein, refers to the production of alcohols from olefins via a carbonylation and a hydrogenation process. Other processes may be used to produce aliphatic alcohols from olefins. Examples of other processes to produce aliphatic alcohols from olefins include, but are not limited to, hydradration, oxidation and hydrolysis, sulfation and hydration, and epoxidation and hydration. The composition of an alcohol product stream may include aliphatic alcohols having an average carbon number ranging from 5 to 31. In an embodiment, an average carbon number of the aliphatic alcohols in an alcohol product stream may range from 7 to 19. In certain embodiments, an average carbon number of the aliphatic alcohols in an alcohol product stream may range from 11 to 18. In some embodiments, an average carbon number of aliphatic alcohols in an alcohol product stream may range from 11 to 14. In other embodiments, an average carbon number of aliphatic alcohols in an alcohol product stream may range from 15 to 18.

In certain embodiments, a first hydrocarbon stream that includes paraffins and olefins may be introduced into a dehydrogenation-isomerization unit. The dehydrogenation-isomerization unit may replace two independent units (e.g., an isomerization unit and a dehydrogenation unit). The dehydrogenation-isomerization unit may dehydrogenate paraffins to olefins and isomerize the resulting olefins and/or initial olefins present in the hydrocarbon stream to branched olefins. In an embodiment, a catalyst may perform the dehydrogenation-isomerization of the hydrocarbons in the first hydrocarbon stream. In certain embodiments, a catalyst may be a single catalyst. The catalyst, in some embodiments, may be a mixture of two catalysts (e.g., a dehydrogenation catalyst and an isomerization catalyst). In other embodiments, two separate catalysts located in different zones or in a stacked bed configuration in one dehydrogenation-isomerization unit may perform the dehydrogenation-isomerisation process. As used herein, "a dehydrogenation-isomerization catalyst" may be one or more catalysts.

In certain embodiments, a dehydrogenation-isomerization unit may have several points of entry to accommodate different process streams. The process streams may be from other processing units and/or storage units. Examples of process streams include, but are not limited to, a diluent hydrocarbon stream, and/or other hydrocarbon streams that include olefins and paraffins derived from other processes. As used herein, "entry into the dehydrogenation-isomerization unit" refers to entry of process streams into the dehydrogenation-isomerization unit through one or more entry points.

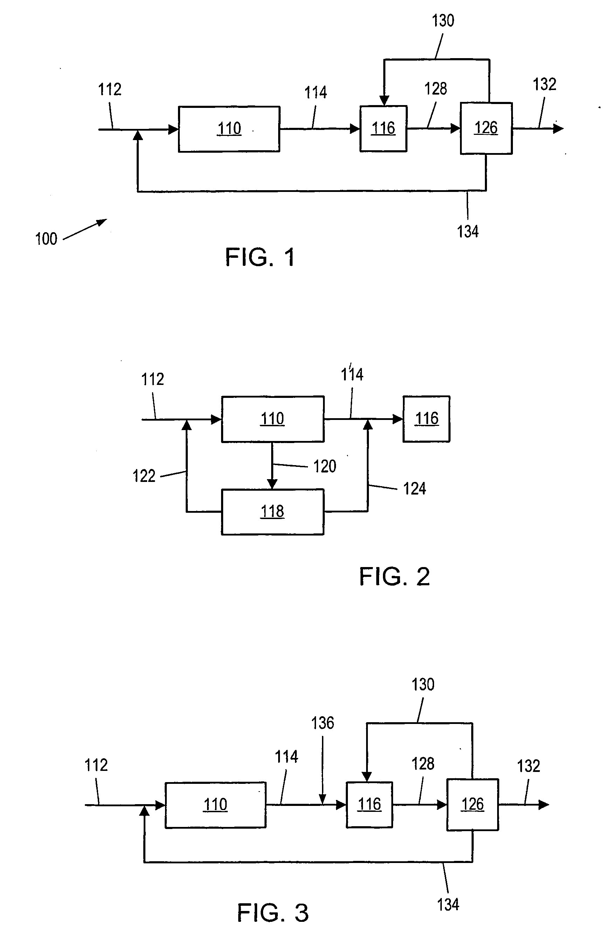

A first hydrocarbon stream, including a mixture of olefins and paraffins, may be introduced into dehydrogenation-isomerization unit 110 via first conduit 112 as depicted for System 100 in FIG. 1. Hydrocarbons in the first hydrocarbon stream may have an average carbon number from 7 to 18. In certain embodiments, hydrocarbons in the first hydrocarbon stream may have an average carbon number from 10 to 17. In some embodiments, hydrocarbons in the first hydrocarbon stream may have an average carbon number from 10 to 13. In other embodiments, hydrocarbons in the first hydrocarbon stream may have an average carbon number from 14 to 17. In some embodiments, a first hydrocarbon stream includes alpha-olefins. The alpha-olefin content of the first hydrocarbon stream may be greater than 70 percent of the total amount of olefins in the first hydrocarbon stream, In certain embodiments a first hydrocarbon stream may be produced from a Fischer-Tropsch process.

In dehydrogenation-isomerization unit 110, at least a portion of the paraffins in the first hydrocarbon stream may be dehydrogenated to olefins. At least a portion of the resulting olefins and at least a portion of the olefins that were already present in the feed stream may be isomerized to produce a second hydrocarbon stream. The isomerization process converts linear olefins (i.e., unbranched olefins) into branched olefins.

The catalyst used for the dehydrogenation-isomerization of the first hydrocarbon stream may be based on a zeolite catalyst modified with one or more metals or metal compounds. The catalyst used in dehydrogenation-isomerization unit 110 to treat the olefins in the first hydrocarbon stream may be effective for skeletally isomerizing linear olefins in the process stream into olefins having an average number of branches per olefin molecule chain greater than 0.7. In certain embodiments, an average number of branches per olefin molecule chain may range from 0.7 to 2.5. In some embodiments, an average number of branches per olefin molecule chain may range from 0.7 to 2.2. In other embodiments, an average number of branches per olefin molecule chain may range from 1.0 to 2.2.

The dehydrogenation-isomerization catalyst may contain a zeolite having at least one channel with a crystallographic free channel diameter greater than 4.2 Å and less than 7 Å, measured at room temperature. As used herein, "channel diameter or size" refers to an effective channel diameter or size for diffusion. The zeolite may have no channels present that have a free channel diameter greater than 7 Å. The catalyst may contain at least one channel having a crystallographic free diameter at the entrance of the channel greater than 4.2 Å and less than 7 Å. The catalyst may not have a channel with a diameter at the entrance, which exceeds the 7 Å upper limit of the range. Zeolites possessing channel diameters greater than 7 Å may be susceptible to undesirable olefin by-products (e.g., aromatization, oligomerization, alkylation, coking). In some embodiments, a zeolite may not contain a channel having a free diameter along either of the x or y planes of greater than 4.2 Å. A small channel size may prevent diffusion of the olefin into and/or out of the channel pore once the olefin becomes branched. A zeolite may have at least one channel with a free diameter of the channel within a range of greater than 4.2 Å and less than 7 Å.

In an embodiment, an olefin molecule, due to its high carbon chain length, may not have to enter into the zeolite channel, diffuse through, and exit the other end of the channel. The rate of branching seen when passing the olefin across the zeolite may not correspond to the theoretical rate of branching if each olefin molecule were to pass through the channels. Most of the olefins may partially penetrate the channel for a distance effective to branch the portion of the chain within the channel and subsequently withdraw from the channel once isomerized. In man embodiment of a method to produce aliphatic alcohols, olefin molecules in a hydrocarbon stream may predominately have a structure which is branched at the ends of the olefin carbon backbone, and substantially linear towards the center of the molecule, (e.g., at least 25 percent of the carbons at the center are unbranched).

In certain embodiments, a zeolite catalyst structure may contain channels having free diameters greater than 4.2 Å and less than 7 Å along both the x and y planes in the [001] view. Zeolites with the specified channel size may be referred to as medium or intermediate channel zeolites and typically have a 10-T member (or puckered 12-T member) ring channel structure in one view and a 9-T member or less (small pore) in another view, if any. There is no limit to channel numbers or orientation (e.g., parallel, non-interconnecting intersections, or interconnecting at any angle) in the zeolite.

Examples of zeolites with a channel size from 4.2 Å to 7.0 Å include molecular sieves, ferrierite, A1PO-31, SAPO-11, SAPO-31, SAPO-41, FU-9, NU-10, NU-23, ZSM-12, ZSM-22, ZSM-23, ZSM-35, ZSM-48, ZSM-50, ZSM-57, SUZ-4A, MeAPO-11, MeAPO-31, MeAPO-41, MeAPSO-11, MeAPSO-31, and MeAPSO-41, MeAPSO-46, ELAPO-11, ELAPO-31, ELAPO-41, ELAPSO-11, ELAPSO-31, and ELAPSO-41, laumontite, cancrinite, offretite, hydrogen form, of stilbite, the magnesium or calcium form of mordenite and partheite. The isotypic structures of the zeolite frameworks, known under other names, may be considered equivalent. Zeolite framework is described by Flanigen et al.; in "Aluminophosphate Molecular Sieves and the Periodic Table," New Developments in Zeolite Science Technology, 1986, Kodansha Ltd., Tokyo, Japan.

Many natural zeolites such as ferrierite, heulandite and stilbite may feature a one-dimensional pore structure with a pore size at or slightly smaller than 4.2 Å in diameter. U.S. Patent No. 4,795,623 to Evans, entitled "Time Effective Method For Preparing Ferrierite" and U.S. Patent No. 4,942,027 to Evans, entitled "Method for Preparing Ferrierite," describe converting channels in natural zeolites to larger channels. Channels in natural zeolites may be converted to zeolites with desired larger channel sizes by removing an associated alkali metal or alkaline earth metal by generally known methods (e.g., ammonium ion exchange, optionally followed by calcination, to yield a zeolite in substantially a hydrogen form). Replacing the associated alkali or alkaline earth metal with the hydrogen form may enlarge the channel diameter. In some embodiments, natural zeolites (e.g., some forms of mordenite) may have a channel size greater than 7 Å. The channel size may be reduced by substituting an alkali metal for larger ions (e.g., a larger alkaline earth metal).

In certain embodiments, zeolites may have a ferrierite isotypic (or homeotypic) framework structure. The prominent structural features of ferrierite found by x-ray crystallography may be parallel channels in the alumino-silicate framework. The parallel channels may have an elliptical cross section. Zeolites having a ferrierite isotypic framework structure are described in European Patent No. 55 529 to Seddon et al., entitled, "Zeolites;" and European Patent No. 103 981 to Whittam, entitled "Zeolites." Zeolites having a ferrierite isotypic framework are also described in U.S. Patent No. 4,016,245 to Plank et al., U.S. Patent No. 4,578,259 to Morimoto et al., entitled "Process For Preparing A Crystalline Aluminosilicate;" entitled "Crystalline Zeolite And Method Of Preparing Same" and U.S. Patent No. 4,375,573 to Young et al., entitled "Selective Production And Reaction of P-Disubstituted Aromatics Over Zeolite ZSM-48."

In an embodiment, a hydrogen form of ferrierite (H-ferrierite) may be considered to be substantially one-dimensional. H-ferrierite may have parallel running channels. H-ferrrierite may have elliptical channels that have free diameters of 4.2 Å by 5.4 Å along the x and y planes in the [001] view. The channels may be large enough to permit entry of a linear olefin and diffusion out of or through the channel of the methyl branched isoolefin. The channels may be small enough to retard coke formation. Methods for preparing various H-ferrierite are described in U.S. Patent No. 5,985,238 to Pasquale et al., entitled "Process For Preparing Ferrierite;" U.S. Patent No. 4,251,499 to Nanne et al., entitled "Process For The Preparation Of Fernerite;" U.S. Patent No. 4,795,623 to Evans, entitled "Time Effective Method For Preparing Ferrierite" and U.S. Patent No. 4,942,027 to Evans, entitled "Method for Preparing Ferrierite."

In certain embodiments, a dehydrogenation-isomerization catalyst may be combined with a refractory oxide that serves as a binder material. Suitable refractory oxides include, but are not limited to, natural clays (e.g., bentonite, montmorillonite, attapulgite, and kaolin), alumina, silica, silica-alumina, hydrated alumina, titania, zirconia or mixtures thereof.

Examples of alumina binders may include, but are not limited to, pseudoboehmite, gamma and bayerite aluminas. Alumina binders may be commercially available (e.g., LaRoche Chemicals manufactures VERSAL® aluminas and Sasol manufactures CATAPAL® aluminas). In an embodiment, high-dispersity alumina powders may be used as alumina binders when extrusion is utilized for catalyst preparation. High-dispersity alumina powders may have a dispersity of greater than 50 percent in an aqueous acid dispersion having an acid content of 0.4-milligram equivalents of acid (acetic) per gram of powder, Such high-dispersity aluminas may be exemplified by CATAPAL® alumina manufactured by Sasol.

A weight ratio of zeolite to binder material may range from 10:90 to 99.5:0.5. In some embodiments, a weight ratio may range from 75:25 to 99:1. In other embodiments, a weight ratio of zeolite to binder material may range from 80:20 to 98:2. In certain embodiments, a weight ratio of zeolite to binder material may range from 85:15 to 95:5 on an anhydrous basis.

In certain embodiments, a dehydrogenation-isomerization catalyst may be prepared with one or more monocarboxylic acids and/or inorganic acids. In addition to the monocarboxylic and/or inorganic acids, at least one organic acid with at least two carboxylic acid groups ("polycarboxylic acid") may be used. Monocarboxylic acids may have a substituted or unsubstituted hydrocarbyl group having 1 to 20 carbon atoms. The hydrocarbyl group may be aliphatic, cyclic or aromatic. Examples of monocarboxylic acids having 1 to 20 carbon atoms include, but are not limited to, acetic acid, formic acid, propionic acid, butyric acid, caproic acid, glycolic acid, lactic acid, hydroxylbutyric acid, hydroxycyclopentanoic acid, salicylic acid, mandelic acid, benzoic acid and fatty acids. Examples of inorganic acids include, but are not limited to, nitric acid, phosphoric acid, sulfuric acid and hydrochloric acid.

The polycarboxylic acid may, in certain embodiments, be an organic acid with two or more carboxylic acid groups attached through a carbon-carbon bond linkage to a hydrocarbon segment The linkage may be at any portion of the hydrocarbon segment. The polycarboxylic acid may have a hydrocarbon segment with less than 10 carbon atoms. The hydrocarbon segment may be aliphatic, cyclic or aromatic. The hydrocarbon segment may have zero carbon atoms for oxalic acid with two carboxylic acid groups attached through the carbon-carbon bond. Examples of the polycarboxylic acids include, but are not limited to, tartaric acid, citric acid, malic acid, oxalic acid, adipic acid, malonic acid, galactaric acid, 1,2-cyclopentane dicarboxylic acid, maleic acid, fumaric acid, itaconic acid, phthalic acid, terephthalic acid, phenylmalonic acid, hydroxyphthalic acid, dihydroxyfumaric acid, tricarballylic acid, benzene-1,3,5-tricarboxylic acid, isocitric acid, mucic acid and glucaric acid. The polycarboxylic acids may be any isomers of the above acids. In some embodiments, the polycarboxylic acids may be any stereoisomers of the above acids. In an embodiment, polycarboxylic acids with at least two carboxylic acid groups and at least one hydroxyl group are used. In an embodiment, citric acid, tartaric acid and malic acid may be used as polycarboxylic acids.

Metals incorporated into a dehydrogenation-isomerization catalyst may be metals that promote the oxidation of coke in the presence of oxygen at a temperature greater than 250 °C and the dehydrogenation of paraffins. "Metal(s)," as used herein, refers to metals of a zero oxidation state and/or higher oxidation states (e.g., metal oxides), As used herein, "coke" refers to a product from thermal degradation of larger molecules into smaller molecules.

Metals used in the dehydrogenation-isomerization catalyst may be transition and rare earth metals. Coke oxidation-promoting metals include, but are not limited to, Groups IB, VB, VIB, VIIB, VIII of the transition metal series of the Periodic Table and/or combinations thereof. In certain embodiments, Pd, Pt, Ni, Co, Mn, Ag, Cr and/or combinations thereof may be used in the dehydrogenation-isomerization catalyst. In other embodiments, metal oxides such as, but not limited to, chrome oxide, iron oxide, noble metals, or mixtures thereof may be used as coke-oxidizing compounds in the catalyst.

An amount of the metal introduced may range from 5 parts per million ("ppm") up to 15 percent by weight. In certain embodiments, an amount of metal may range from 5 ppm to 10 percent by weight. In some embodiments, an amount of metal may range from 5 ppm to 5 percent by weight.

Noble metals (e.g., platinum and/or palladium) may be used in smaller amounts of metals than other metals incorporated into a zeolite and/or binder. "Noble metals," as used herein, refers to metals of the group that includes platinum, palladium, indium, ruthenium, osmium and rhodium. In certain embodiments, an amount of noble metals may range from 5 ppm to 2 percent by weight, basis metal, of the final catalyst. In some embodiments, an amount of noble metals may range from 5 ppm to 1000 ppm, basis metal, of the final catalyst. In other embodiments, an amount of noble metal(s) may range from 5 ppm to 3000 ppm, basis metal, of the final catalyst. An amount of noble metal(s) used in a dehydrogenation-isomerization catalyst may, in certain embodiments, range from 5 ppm to 2000 ppm by weight, basis metal, of the final catalyst. An amount of noble metal(s) sufficient to promote regeneration without deteriorating the performance of the catalyst may be in the range from 30 ppm to 100 ppm. Higher amounts of platinum and/or palladium (e.g., greater than 2% by weight) may have an adverse effect on the catalyst (e.g., run life, olefin isomerization activity, selectivity).

In an embodiment, zeolite powder and alumina powder may be mixed (e.g., mulled) with water and one or more metal compounds of the catalyst. The resulting mixture may be formed into a pellet Catalysts prepared by mulling may have superior olefin isomerization performance over catalysts prepared by impregnation. The term "mulling," as used herein, refers to mixing of powders to which sufficient water has been added to form a thick paste and wherein the mixing is accompanied by concomitant shearing of the paste. Commercially available mullers such as the Lancaster Mix Muller and the Simpson Mix Muller may be used.

The pellet may be formed, in some embodiments, by extrusion. One or more peptizing acid (e.g., nitric acid, acetic acid, citric acid or mixtures thereof) may be added to the mixture and optional extrusion aids such as cellulose derivatives (e.g., METHOCEL^{®}F4M, hydroxypropyl methylcellulose, manufactured by The Dow Chemical Company) may be utilized. The amounts of peptizing acid used may be determined by routine experimentation to provide a plastic, extrudable material. The term "pellets," as used herein, refers to any shape or form of consolidated materials.

In certain embodiments, a noble metal such as platinum and/or palladium may be added to the zeolitic catalyst after pelletization. Common metal incorporation methods known to those skilled in the art (e.g., impregnation, noble metal ion exchange and co-mulling) may be used to produce a working catalyst useful in dehydrogenation-isomerization of paraffins. The addition of noble metals to the catalyst may aid in the dehydrogenation reaction of paraffins. Pellets containing noble metals may be calcined at a temperature range from 250 °C to 700 °C. In certain embodiments, a calcination temperature may range from 300°C to 600 °C. In some embodiments, a calcination temperature may range from 450 °C to 525 °C.

The dehydrogenation-isomerization catalyst may be contacted with the first hydrocarbon stream in dehydrogenation-isomerization unit 110 under a variety of conditions to dehydrogenate at least a portion of the paraffins to olefins and isomerize at least a portion of the resulting olefins. In dehydrogenation-isomerization unit 110, reaction temperatures may range from 300 °C to 700 °C. A reaction temperature, in some embodiments, may range from 350 °C to 550 °C. A total pressure of dehydrogenation-isomerization unit 110 during the reaction may range from 0.010 atmosphere (1 kPa) to 25.0 atmospheres (2534 kPa). In an embodiment, a total pressure of dehydrogenation-isomerization unit 110 during the reaction may range from 0.010 atmosphere (1 kPa) to 15.0 atmospheres (1520 kPa). In other embodiments, a total pressure of dehydrogenation-isomerization unit 110 during the reaction may range from 1 atmosphere (101 kPa) to 5.0 atmospheres (507 kPa). In order to prevent coking, hydrogen may be fed together with the first hydrocarbon stream. Hydrogen gas and paraffins present in the first hydrocarbon stream may be fed at a hydrogen gas to paraffin molar ratio in the range from 0.1 to 20. In certain embodiments, a hydrogen gas to paraffin molar ratio may be in the range from 1 to 10.

Residence time in dehydrogenation-isomerization unit 110 may be selected such that conversion level of the paraffins to olefins may be kept below 40 mole percent. In an embodiment, a conversion level ranges from 5 mole percent to 30 mole percent. By keeping the conversion level low, side reactions (e.g., diene formation and cyclization reactions) may be minimized. Olefin conversion may be increased by varying the reaction conditions (e.g., temperature, residence time) as long as side reactions remain below acceptable limits. Olefins produced in dehydrogenation-isomerization unit 110 may have a higher degree of branching than a paraffin feed to the dehydrogenation-isomerization unit. It should be understood that the concentration of olefins produced via dehydrogenation-isomerization unit 110 may be limited by the thermodynamic equilibrium of olefins and paraffins at the reaction temperature. The conditions for olefin isomerization dehydrogenation-isomerization 110 may be controlled such that the number of carbon atoms in the olefins prior to and subsequent to the isomerization conditions is substantially the same.

Branched olefins produced in dehydrogenation-isomerization unit 110 may include methyl, ethyl and/or longer carbon chain branches. Hydrogen Nuclear Magnetic Resonance (¹H NMR) analysis of the isomerized olefin composition may be performed. Branched olefins may include quaternary and/or tertiary aliphatic carbons. In certain embodiments, an amount of quaternary aliphatic carbons produced in a unit in which olefin isomerization occurs may be minimized. ¹H NMR analysis of the olefins may indicate the extent of isomerization of the olefins in the hydrocarbon stream. ¹H NMR analysis may be capable of differentiating a wide range of olefin structures. The presence of quaternary carbon atoms may be determined using carbon 13 (¹³C) NMR techniques. The type of branching (e.g., methyl, ethyl, propyl or larger groups) may be determined by hydrogenation of the olefin mixture and then ¹³C NMR analysis of the hydrogenated olefin solution.

Aliphatic branches, as used herein, refer to branches on non-olefinic carbons. Olefinic branches, as used herein, refer to branches on olefinic carbons. The total number of branches on a double bond may be determined by summation of the individual contributions of the various assayed olefin units. Olefin units include vinyl, di-substituted, tri-substituted, vinylidene and/ or tetra-substituted olefins. The amount and type of olefin may vary with process stream composition and isomerization reaction conditions. In an embodiment, an amount of tetra-substituted olefin produced may be low.

In an embodiment, an average number of branches per olefin molecule present in the produced branched olefin composition may be greater than 0.7. In certain embodiments, an average number of branches per olefin molecule present in the branched olefin composition is from 0.7 to 2.5. In some embodiments, an average number of branches per olefin molecule present in the branched olefin composition is from 0.7 to 2.2. In certain embodiments, an average number of branches per olefin molecule present in the branched olefin composition is from 1.0 to 2.2. The degree of branching in the product may be controlled by controlling process conditions used in a unit in which olefin isomerization occurs. For example, high reaction temperatures and lower feed rates may result in a higher degree of branching. Methyl branches may represent between 20 percent to 99 percent of the total number of branches present in the olefin molecules, In some embodiments, methyl branches may represent greater than 50 percent of the total number of branches in the olefin molecules. The number of ethyl branches in the olefin molecules may represent, in certain embodiments, less than 30 percent of the total number of branches. In other embodiments, a number of ethyl branches, if present, may be between 0.1 percent and 2 percent of the total number of branches. Branches other than methyl or ethyl, if present, may be less than 5 percent of the total number of branches.

Aliphatic quaternary carbon atoms present in the branched olefin composition may be less than 2 percent of the carbon atoms present. In an embodiment, a number of aliphatic quaternary carbon atoms present is less than 1 percent of the carbon atoms present. For applications in which biodegradability is important, the number of aliphatic quaternary carbon atoms may be less than 0.5 percent of the carbon atoms present. In an embodiment, a number of aliphatic quaternary carbon atoms is less than 0.3 percent of the carbon atoms present. In other embodiments, a number of aliphatic quaternary carbon atoms present in the branched olefin composition is between 0.01 percent and 0.3 percent of the aliphatic carbon atoms present.

A second hydrocarbon stream may exit dehydrogenation-isomerization unit 110 and be transferred to other processing units (e.g., a hydroformylation unit, separation units, an alkylation units) via second conduit 114. At least a portion of the second hydrocarbon stream may exit dehydrogenation-isomerization unit 110 and be introduced into hydroformylation unit 116 via second conduit 114. In hydroformylation unit 116, at least a portion of the olefins in the second hydrocarbon stream may be converted to alcohols. At least a portion of the produced alcohols and at least a portion of the unreacted components of the second hydrocarbon stream may form a hydroformylation reaction stream.

In an embodiment, olefins may be separated, if desired, from the second hydrocarbon stream through techniques generally known in the art (e.g., distillation, molecular sieves, extraction, adsorption, adsorption/desorption, and/or membranes). Separation of at least a portion of the branched olefins from the linear olefins and paraffins, may increase the concentration of branched olefins entering the hydroformylation unit. In addition, separation of at least a portion of the branched olefins from the linear olefins and paraffins may influence the ratio of linear to branched olefins produced in the hydroformylation unit.

A second hydrocarbon stream may exit dehydrogenation-isomerization unit 110 and enter separation unit 118 via separation conduit 120 as depicted in FIG. 2. Separation unit 118 may produce at least two streams, a branched olefins stream and a linear olefins and paraffins stream. In separation unit 118, the second hydrocarbon stream may be contacted with organic and/or inorganic molecular sieves (e.g., zeolite or urea) with the correct pore size for branched olefins and/or linear olefins and paraffins. Subsequent desorption,(e.g., solvent desorption) of at least a portion of the branched olefins and/or at least a portion of the linear olefins and paraffins from the molecular sieves may produce at least two streams (e.g., a branched olefins stream and a linear olefins and paraffins stream).

Separation unit 118 may include a fixed bed containing adsorbent for separation of the second hydrocarbon stream to produce a branched olefin and paraffins stream and a linear olefins and paraffins stream. Separation temperatures in separation unit 118 may range from 100 °C to 400 °C. In some embodiments, separation temperatures may range from 180 °C to 380 °C. Separation in separation unit 118 may be conducted at a pressure ranging from 2 atmospheres (202 kPa) to 7 atmospheres (710kPa). In some embodiments, a pretreatment of a second hydrocarbon stream may be performed to prevent adsorbent poisoning. An example of an adsorption/desorption process is a Molex process using Sorbex® separations technology (UOP process, UOP, Des Plaines, IL). Adsorption/desorption processes are described in U.S. Patent No. 6,225,518 to Sohn et al., entitled "Olefinic Hydrocarbon Separation Process;" U.S. Patent No. 5,292,990 to Kantner et al., entitled, "Zeolite Compositions For Use in Olefinic Separations" and U.S. Patent No. 5,276,246 to McCulloch et al., entitled "Process For Separating Normal Olefins From Non-Normal Olefins."

At least a portion of the linear olefins and paraffins stream may be transported to other processing units and/or stored on site. In an embodiment, at least a portion of the linear olefins and paraffins stream may be combined with first hydrocarbon stream in first conduit 112 via linear olefin and paraffin recycle conduit 122. The combined stream may enter dehydrogenation-isomerization unit 110 via first conduit 112 to continue the process to produce aliphatic alcohols. In some embodiments, a linear olefins and paraffins stream may be introduced directly into dehydrogenation-isomerization unit 110.

At least a portion of the branched olefins stream may be transported and utilized in other processing streams and/or stored on site via branched olefins conduit 124. In some embodiments, at least a portion of a branched olefins stream may exit separation unit 118 and be introduced into second conduit 114 via branched olefins conduit 124. In other embodiments, at least a portion of a branched olefins stream may exit separation unit 118 and be introduced directly into a hydroformylation unit.

A second hydrocarbon stream may exit dehydrogenation-isomerization unit and enter hydroformylation unit 116 via second conduit 114 as depicted in FIGS. 1 and 2. Hydroformylation unit 116 may have several points of entry to accommodate entry of additional process streams. As used herein, "stream entering into the hydroformylation unit" is defined as the entry of process streams into the hydroformylation unit through one or more entry points. Examples of such process streams include, but are not limited to, additional streams from dehydrogenation-isomerization unit 110, a diluent hydrocarbon stream, gases and/or other hydrocarbon streams that include olefins and paraffins derived from other processes.

In a hydroformylation process, olefins are converted to aldehydes, alcohols or a combination thereof by reaction of at least a portion of the olefins with carbon monoxide and hydrogen according to an Oxo process. As used herein, an "Oxo process" refers to the reaction of an olefin with carbon monoxide and hydrogen in the presence of a metal catalyst (e.g., a cobalt catalyst) to produce an alcohol containing one more carbon atom than the starting olefin. In other hydroformylation processes, a "modified Oxo process" is used. As used herein, a "modified Oxo process" refers to an Oxo process that uses a phosphine, phosphite, arsine or pyridine ligand modified cobalt or rhodium catalyst. Preparation and use of modified Oxo catalysts are described in U.S. Patent No. 3,231, 621, to Slaugh, entitled "Reaction Rates In Catalytic Hydroformylation"; U.S. Patent No. 3,239,566 to Slaugh et al., entitled "Hydroformylation Of Olefins;" U.S. Patent No. 3,239,569 to Slaugh et al., entitled "Hydroformylation Of Olefins;" U.S. Patent No. 3,239,570 to Slaugh et al., entitled "Hydroformylation Of Olefins;" U.S. Patent No. 3,239,571 to Slaugh et al., entitled "Hydroformylation Of Olefins;" U.S. Patent No. 3,400,163 to Mason et al., entitled "Bicyclic Heterocyclic Sec- And Tert-Phosphines;" U.S. Patent No. 3,420,898 to Van Winkle et al., entitled "Single Stage Hydroformylation Of Olefins To Alcohols Single Stage Hydroformylation Of Olefins To Alcohols;" U.S. Patent No. 3,440,291 to Van Winkle et al., entitled "Single Stage Hydroformylation Of Olefins To Alcohols;" U.S. Patent No. 3,448,157 to Slaugh et al., entitled "Hydroformylation Of Olefins;" U.S. Patent No. 3,488,158 to Slaugh et al., entitled "Hydroformylation Of Olefins;" U.S. Patent No. 3,496,203 to Morris et al., entitled "Tertiary Organophosphine-Cobalt-Carbonyl Complexes;" U.S. Patent No. 3,496,204 to Morris et al., entitled "Tertiary Organophosphine-Cobalt-Carbonyl Complexes;" U.S. Patent No, 3,501,515 to Van Winkle et al., entitled "Bicyclic Heterocyclic Terteriary Phosphine Cobalt-Carbonyl Complexes"; U.S. Patent No. 3,527,818 to Mason et al., entitled "Oxo Alcohols Using Catalysts Comprising Ditertiary Phosphines;" U.S. Patent Application Serial No. 10/075682, entitled "A Process For Preparing A Branched Olefin, A Method Of Using The Branched Olefin For Making A Surfactant, and a Surfactant" and in U.S. Patent Application Serial No. 10/167209 entitled "Process for the Preparation Of A Highly Linear Alcohol Composition." Methods of alcohol production are also described by Othmer, in "Encyclopedia of Chemical Technology" 2000, Fourth Edition; and by Wickson, in "Monohydric Alcohols; Manufacture, Applications and Chemistry" Ed. Am. Chem. Soc. 1981.

A hydroformylation catalyst used in hydroformylation unit 116 may include a metal from Group VIII of the Periodic Table. Examples of Groups VIII metals include cobalt, rhodium, nickel, palladium or platinum. The Group VIII metal may be used as a complex compound. A complex compound may be a Group VIII metal combined with a ligand. Examples of ligands include, but are not limited to, a phosphine, phosphite, arsine, stibine or pyridine ligand. Examples of hydroformylation catalysts include, but are not limited to, cobalt hydrocarbonyl catalyst, cobalt-phosphine ligand catalyst, rhodium-phosphine ligand catalyst or combinations thereof.

A source of carbon and hydrogen for a hydroformylation process in hydroformylation unit 116 may be a gas. Examples of gases include, but are not limited to, carbon monoxide, hydrogen or synthesis gas. A ratio of carbon monoxide to hydrogen applied in hydroformylation unit 116 may be in a range from 1.0 to 5.0. In certain embodiments, a hydrogen to carbon monoxide molar ratio may be in a range from 1.5 to 2.5.

Synthesis gas that contains hydrogen and carbon monoxide in a molar ratio from 1 to 2.5 may be used in hydroformylation unit 116. In other embodiments, synthesis gas that contains hydrogen and carbon monoxide in a molar ratio from 1.0 to 10.0 may be used. In other embodiments, synthesis gas that contains a hydrogen and carbon monoxide molar ratio from 1.5 to 2.5 may be used as a source of carbon and hydrogen. It should be understood that the gas feed may be a mixture of carbon monoxide and hydrogen gases only, synthesis gas only or combinations thereof.

In hydroformylation unit 116, olefins in the second hydrocarbon stream may be hydroformylated using a continuous, semi-continuous or batch process. In case of a continuous mode of operation, the liquid hourly space velocities may be in the range of 0.1 h⁻¹ to 10 h⁻¹. When operating hydroformylation unit 116 as a batch process, reaction times may vary from 0.1 hours to 10 hours or even longer.

Reaction temperatures in hydroformylation unit 116 may range from 100 °C to 300 °C. In certain embodiments, reaction temperatures in the hydroformylation unit ranging from 125 °C to 250 °C may be used. Pressure in hydroformylation unit 116 may range from 1 atmosphere (101 kPa) to 300 atmospheres (30398 kPa). In an embodiment, a pressure from 20 (2027 kPa) to 150 atmospheres (15199 kPa) may be used. An amount of catalyst relative to the amount of olefin to be hydroformylated may vary. Typical molar ratios of catalyst to olefin in the second hydrocarbon stream may range from 1:1000 to 10:1. A ratio of between 1:10 and 5:1 may be used in certain embodiments. In an embodiment, a second stream may be added to hydroformylation unit 116 to control reaction conditions. The second stream may include solvents that do not interfere substantially with the desired reaction. Examples of such solvents include, but are not limited to, alcohols, ethers, acetonitrile, sulfolane and paraffins.

Mono-alcohol selectivities of at least 90 percent and even of at least 92 percent may be achieved in hydroformylation unit 116. In addition, olefin conversions to aliphatic alcohols may range from 50 percent by weight to greater than 95 percent by weight. In certain embodiments, olefin conversion to aliphatic alcohols may be greater than 75 percent by weight. In some embodiments, olefin conversion to aliphatic alcohols may be greater than 99 percent by weight.

Isolation of aliphatic alcohols produced from the hydroformylation reaction product stream may be achieved by generally known methods. In an embodiment, isolation of the aliphatic alcohols includes subjecting the produced aliphatic alcohols to a first distillation, a saponification, a water washing treatment and a second distillation.

The hydroformylation reaction mixture stream may enter separator 126 via third conduit 128. In separator 126, the hydroformylation reaction product stream may be subjected to a first distillation step (e.g., flash distillation or a short path distillation). In an embodiment, a short path distillation may be used to produce at least two streams, a bottom stream and a top stream. At least a portion of the bottom stream may be recycled to hydroformylation unit 116 via bottom stream recycle conduit 130, in certain embodiments. The top stream may include, but is not limited to, paraffins, unreacted olefins and a crude aliphatic alcohol product.

In an embodiment, a top stream may be subjected to a saponification treatment to remove any acids and esters present in the stream. Saponification may be performed by contacting the top stream with an aqueous solution of a hydroxide base (e.g., sodium hydroxide or potassium hydroxide) at elevated temperatures with agitation. The saponification may be carried out by contacting the top stream with an aqueous 0.5 percent to 10 percent hydroxide base solution at a crude alcohol/water ratio of 10:1 to 1:1. The amount of hydroxide base used may depend on an estimated amount of esters and acids present.

Saponification of the top stream may be carried out batch-wise or continuously. The top stream may be subjected to one or more saponification processes. Saponification reaction temperatures may be from 40 °C to 99 °C. In an embodiment, saponification temperatures may range from 60 °C to 95 °C. Mixing of the top stream with the basic water layer may be performed during the saponification reaction. Separation of the top stream from the basic water layer may be performed using known methods. The top stream may be subjected to a water wash after separation to remove any sodium salts present. The top stream may be separated using generally known techniques (e.g., fractional distillation) to produce at least two streams, a crude alcohol product stream and a paraffins and unreacted olefins stream. As used herein, "fractional distillation" refers to the distillation of liquids and subsequent collection of fractions of liquids determined by boiling point. The paraffins and unreacted olefins stream may be recycled, transported to other units for processing, stored on site, transported offsite and/or sold.

In certain embodiments, a crude aliphatic alcohol product stream may contain unwanted by-products (e.g., aldehydes, hemi-acetals). The by-products may be removed by subjecting the crude alcohol product stream to a hydrofinishing treatment step to produce an aliphatic alcohol product stream. "Hydrofinishing," as used herein, refers to, a hydrogenation reaction carried out under relatively mild conditions. Hydrofinishing may be carried out using conventional hydrogenation processes. Conventional hydrogenation processes may include passing the crude alcohol feed together with a flow of hydrogen over a bed of a suitable hydrogenation catalyst. The aliphatic alcohol product stream may include greater than 50 percent by weight of the produced aliphatic alcohols. In some embodiments, the aliphatic alcohol product stream may include greater than 80 percent by weight of the produced aliphatic alcohols. In other embodiments, the aliphatic alcohol product stream may include greater than 95 percent by weight of the produced aliphatic alcohols. The aliphatic alcohol product stream may include branched aliphatic primary alcohols. The resulting aliphatic alcohols in the aliphatic alcohol product, stream may be sold commercially, transported off-site, stored on site and/or used in other processing units via product conduit 132.

The composition of an aliphatic alcohol product stream may include hydrocarbons with an average carbon number ranging from 8 to 19. In an embodiment, an average carbon number of the hydrocarbons in aliphatic alcohol product stream may range from 10 to 17. The aliphatic alcohol product stream may include branched primary alcohols. The branched primary alcohol product may be suitable for the manufacture of anionic, nonionic and cationic surfactants. In some embodiments, branched primary alcohol products may be used as the precursor for the manufacture of anionic sulfates, including aliphatic sulfates and oxyalkyl sulfates and oxyalkyl alcohols.

Aliphatic alcohols may have slightly higher aliphatic branching and slightly higher number of quaternary carbons as the olefin precursor. In some embodiments, aliphatic branching may include methyl and/or ethyl branches. In other embodiments, aliphatic branching may include methyl, ethyl and higher aliphatic branching. In certain embodiments, a number of quaternary carbon atoms in the aliphatic alcohol product may be less than 0.5. In other embodiments, a number of quaternary carbon atoms in the aliphatic alcohol product may be less than 0.3. Branching of the alcohol product may be determined by ¹H NMR analysis. The number of quaternary carbon atoms may be determined by ¹³C NMR. A ¹³C NMR method for determining quaternary carbon atoms for branched aliphatic alcohols is described in U.S. Patent No. 6,150,322 to Singleton et al., entitled, "Highly Branched Primary Alcohol Compositions and Biodegradable Detergents Made Therefrom."

In certain embodiments, at least a portion of the paraffins and unreacted olefins stream may be combined with the first hydrocarbon stream in first conduit 112 to produce a combined stream via fourth conduit 134. The combined stream may be introduced into dehydrogenation-isomerization unit 110 via first conduit 112. At least a portion of the olefins in the combined stream may be isomerized to branched olefins. In some embodiments, at least a portion of the paraffins and unreacted olefins stream is introduced directly into dehydrogenation-isomerization unit 110 via one or more entry points. Because the paraffins and unreacted olefins stream containing paraffins and unreacted olefins may be recycled to dehydrogenation-isomerization unit 110 as one stream, the process may be more efficient, resulting in an overall higher throughput. The higher throughput will increase the overall yield of the aliphatic alcohols.

In some embodiments, an olefins and paraffins concentration in hydroformylation unit 116 may be adjusted depending on the source of the olefin stream entering the hydroformylation unit. A third hydrocarbon stream may be added upstream of hydroformylation unit 116 to produce a combined stream. In other embodiments, a third hydrocarbon stream may be introduced directly into hydroformylation unit 116 through one or more points. A third hydrocarbon stream may be introduced into second conduit 114 via fifth conduit 136 to produce a combined stream as depicted in FIG. 3. The combined stream may enter hydroformylation unit 116 via second conduit 114 to continue the process to produce aliphatic alcohols.

The third hydrocarbon stream may be from the same source as the first hydrocarbon stream. In some embodiments, a third hydrocarbon stream may be a hydrocarbon stream that includes olefins, paraffins, and/or hydrocarbon solvents derived from another source. The third hydrocarbon stream may include olefins and paraffins. In certain embodiments, an average carbon number of the hydrocarbons in the third hydrocarbon stream ranges from 7 to 18. In some embodiments, a paraffin content of the third hydrocarbon stream may be between 60 percent and 90 percent by weight. In other embodiments, a paraffin content of the third hydrocarbon stream may be greater than 90 percent by weight.

In an embodiment, an olefin content of a third hydrocarbon stream ranges between 1 percent and 99 percent relative to the total hydrocarbon content. In certain embodiments, an olefin content of the third hydrocarbon stream may be between 45 percent and 99 percent by weight. In other embodiments, an olefin concentration of the third hydrocarbon stream may be greater than 80 percent by weight.

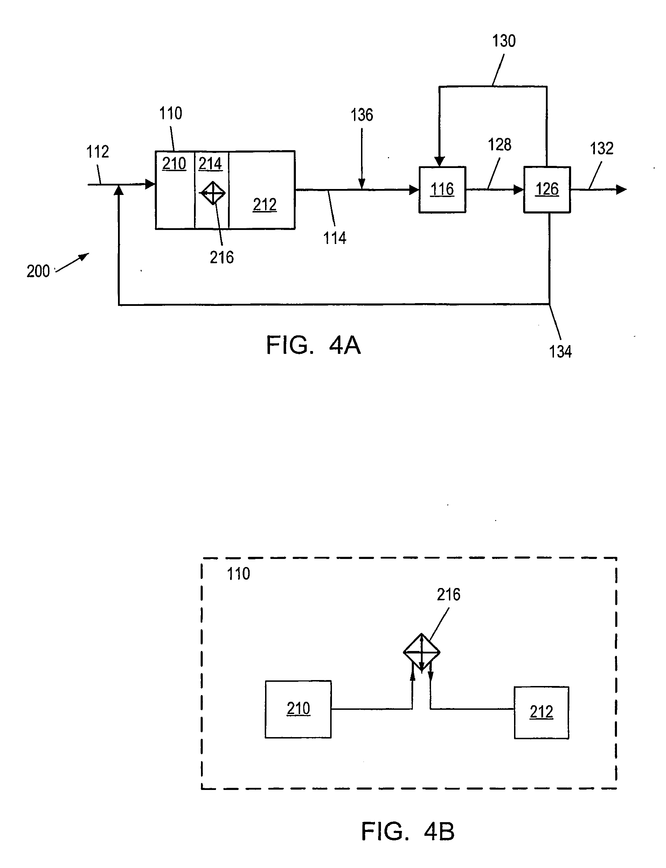

In certain embodiments, dehydrogenation-isomerization unit 110 may be separated into a plurality of zones to control reaction temperatures and/or prevent unwanted side reactions (e.g., diene formation and/or cyclization reactions). A first hydrocarbon stream containing paraffins and unreacted olefins may be introduced into dehydrogenation-isomerization unit 110 via first conduit 112 as depicted for System 200 in FIG. 4A. In some embodiments, a first hydrocarbon stream includes alpha-olefins. Hydrocarbons in the first hydrocarbon stream may have an average carbon number from 7 to 18. In other embodiments, hydrocarbons in the first hydrocarbon stream may have an average carbon number from 10 to 17. In some embodiments, hydrocarbons in the first hydrocarbon stream may have an average carbon number from 14 to 17. In certain embodiments, hydrocarbons in the first hydrocarbon stream may have an average carbon number from 10 to 13. An alpha-olefin content of the first hydrocarbon stream may be greater than 70 percent of the total amount of olefins in the first hydrocarbon stream. In certain embodiments, a first hydrocarbon stream is produced from a Fischer-Tropsch process. Dehydrogenation-isomerization unit 110 may be divided into a plurality of zones. The plurality of zones may include, but is not limited to, a first reaction zone, a transition zone and a second reaction zone. In first reaction zone 210, at least a portion of the paraffins in the first hydrocarbon stream may be dehydrogenated to olefins to produce an olefinic stream. The process stream may then pass into second reaction zone 212. In second reaction zone 212, at least a portion of the olefins in the process stream may be isomerized to branched olefins to produce a second hydrocarbon stream.

In first reaction zone 210, the dehydrogenation catalyst may be selected from a wide range of catalyst types. For example, the catalyst may be based on a metal or metal compound deposited on a porous support. The metal or metal compound may be selected from, but is not limited to, chrome oxide, iron oxide and noble metals.

Techniques of preparing catalysts, for performing the dehydrogenation step and for performing associated separation steps are known in the art. For example, suitable procedures for preparing catalysts and performing the dehydrogenation step are described in U.S. Patent No. 5,012,021 to Vora et al., entitled "Process For the Production of Alkyl Aromatic Hydrocarbons Using Solid Catalysts," U.S. Patent No. 3,274,287 to Moore et al., entitled "Hydrocarbon Conversion Process and Catalyst;" U.S. Patent No. 3,315,007 to Abell et al., entitled "Dehydrogenation of Saturated Hydrocarbons Over Noble-Metal Catalyst;" U.S. Patent No. 3,315,008 to Abell et aL, entitled "Dehydrogenation of Saturated Hydrocarbons Over Noble-Metal Catalyst;" U.S. Patent No. 3,745,112 to Rausch, entitled "Platinum-Tin Uniformly Dispersed Hydrocarbon Conversion Catalyst and Process;" U.S. Patent No. 4,506,032 to Imai et al., entitled "Dehydrogenation Catalyst Composition" and U.S. Patent No. 4,430,517 to Imai et al., entitled "Dehydrogenation Process Using a Catalytic Composition."

Reaction temperatures in first reaction zone 210 may range from 300 °C to 600 °C. In some embodiments, a reaction temperature in first reaction zone 210 may range from 450 °C to 550 °C. A total pressure in first reaction zone 210 may range from 0.010 atmosphere (1 kPa) to 25.0 atmospheres (2534 kPa). In certain embodiments, total pressure in first reaction zone 210 may range from 0.010 atmospheres (1 kPa) to 15.0 atmospheres (1520 kPa). In some embodiments, hydrogen may be fed together with the unreacted first hydrocarbon stream in order to prevent coking. Hydrogen and paraffins present in the unreacted first hydrocarbon stream may be fed at a hydrogen to paraffin molar ratio in a range from 0.1 to 20. In an embodiment, a hydrogen to paraffin molar ratio may be in a range of 1 to 10.

Residence time in first reaction zone 210 may be selected such that a conversion level of the paraffins to olefins is below 50 mole percent. In certain embodiments, a conversion level of the paraffins to olefins may be kept in a range from 10 mole percent to about 20 mole percent. By keeping the conversion level low, side reactions (e.g., diene formation and cyclization reactions) may be presented. In some embodiments, an olefinic hydrocarbon stream may exit first reaction zone 210, pass through transition zone 214 and enter second reaction zone 212. Transition zone 214 may include heat exchanger 216. Heat exchanger 216 may reduce the temperature of the olefinic hydrocarbon stream. In an embodiment, first reaction zone 210 and second reaction zone 212 in dehydrogenation-isomerization unit 110 may be separate units, as depicted in FIG. 4B, with heat exchanger 216 positioned between the two units.

After the olefinic hydrocarbon stream enters second reaction zone 212, at least a portion of the olefins are isomerized to branched olefins to produce a second hydrocarbon stream. The composition and level of branching of the second hydrocarbon stream may be performed by ¹H NMR analysis. In an embodiment, an olefinic stream may exit first reaction zone 210 and directly enter second reaction zone 212 where at least a portion of the olefins in the olefinic stream are isomerized to branched olefins.

The catalyst used for isomerization of the olefins to branched olefins may be the same as described in U. S. Patent No. 5,648,584 to Murray, entitled "Process for Isomerizing Linear Olefins to Isoolefins" and U.S. Patent No. 5,648,585 to Murray et al., entitled "Process for Isomerizing Linear Olefins to Isoolefins."

In an embodiment, linear olefins in a first hydrocarbon stream are isomerized in second reaction zone 212 by contacting at least a portion of the olefinic stream with a zeolite catalyst The zeolite catalyst may have at least one channel with a crystallographic free channel diameter ranging from greater than 4.2 Å to less than 7 Å. The zeolite catalyst may have an elliptical pore size large enough to permit entry of a linear olefin and at least partial diffusion of a branched olefin, The pore size of the zeolite catalyst may also be small enough to retard coke formation.

Temperatures in second reaction zone 212 may be from 200 °C to 500 °C to isomerize linear olefins to branched olefins. In some embodiments, reaction temperatures in the first reaction zone and the second reaction zone are substantially the same. In such embodiments, use of a heat exchanger is not required. Typically, however, the reaction temperature of second reaction zone 212 is less than the reaction temperature of the first reaction zone. The use of a heat exchanger lowers the temperature of the stream leaving the first reaction zone to the appropriate temperature for reaction in the second reaction zone. Hydrocarbon partial pressure in the second reaction zone may range from 0.1 atmosphere (10 kPa) to 10 atmospheres (1013 KPa).

In some embodiments, the second hydrocarbon stream may exit a second reaction zone and enter a separation unit. In the separation unit, branched olefins may be separated from linear olefins and paraffins as previously described with regard to FIG. 2. The second hydrocarbon stream may exit second reaction zone 212 via second conduit 114 and enter hydroformylation unit 116 as depicted in FIG. 4A. At least a portion of the olefins in the second hydrocarbon stream may be hydroformylated to produce a hydroformylation reaction stream as described for System 100. At least a portion of the hydroformylation reaction stream may be separated into a bottom stream and a top stream using generally known methods. The crude aliphatic alcohol product stream may be further purified as described for System 100 to produce a paraffins and unreacted olefins stream and an aliphatic alcohol product stream. The aliphatic alcohol product stream may include branched aliphatic alcohols (e.g., branched primary aliphatic alcohols). The aliphatic alcohol product stream may be recycled, transported to other processing units, sold, and/or transported to storage vessels.

The hydroformylation reaction mixture stream may enter separator 126 via third conduit 128. In separator 126, at least three streams, a bottom stream, a paraffins and unreacted olefins stream and an aliphatic alcohol product stream, may be produced using techniques previously described for System 100. At least a portion of the bottom steam may be recycled to the hydroformylation unit via bottom stream recycle conduit 130. At least a portion of the paraffins and unreacted olefins stream may be recycled combined with other process streams, transported to and/or stored on site. The aliphatic alcohol product stream may be transported via product conduit 132 to be stored on site, sold commercially, transported off-site and/or utilized in other processing units.

In an embodiment, at least a portion of the paraffins and unreacted olefins stream may be combined With the first hydrocarbon stream to produce a combined hydrocarbon stream via fourth conduit 134. The combined hydrocarbon stream may enter first reaction zone 210 and undergo the dehydrogenation-isomerization process and hydroformylation process to produce aliphatic alcohols. By recycling the paraffins and unreacted olefins stream, the yield of product may be maximized. In an embodiment, a paraffins and unreacted olefins stream may directly enter dehydrogenation-isomerization unit 110 through one or more points of entry.

In some embodiments, an olefins and paraffins concentration in hydroformylation unit 116 may be adjusted depending on the source of the olefin stream entering the hydroformylation unit as previously described for System 100. At least a portion of a third hydrocarbon stream may be introduced into second conduit 114 upstream of hydroformylation unit 116 via fifth conduit 136. The combined stream may be introduced into hydroformylation unit 116 via second conduit 114. At least a portion of the olefins in the combined stream may be hydroformylated to produce aliphatic alcohols. In an embodiment, a third hydrocarbon stream may be introduced directly into hydroformylation unit 116 through one or more points of entry.

A third hydrocarbon stream may be used to optimize the olefin concentration in hydroformylation unit 116 at a concentration sufficient to maximize hydroformylation of the olefin. In addition, the third hydrocarbon may optimize the ratio of linear to branched aliphatic groups in the aliphatic alcohol. The third hydrocarbon stream may be, but is not limited to, a hydrocarbon stream containing olefins, paraffins and/or hydrocarbon solvents.

In an embodiment, a third hydrocarbon stream includes a paraffin content of between 50 percent and 99 percent relative to the total hydrocarbon content. In certain embodiments, a paraffin content of the third hydrocarbon stream ranges between 60 percent and 90 percent relative to the total hydrocarbon content. In other embodiments, a paraffin content of the third hydrocarbon stream is greater than 80 percent relative to the total hydrocarbon content.

In an embodiment, an olefin content of a third hydrocarbon stream ranges between 1 percent and 99 percent relative to the total hydrocarbon content. In other embodiments, an olefin content of a third hydrocarbon stream may be greater than 80 percent relative to the total hydrocarbon stream.

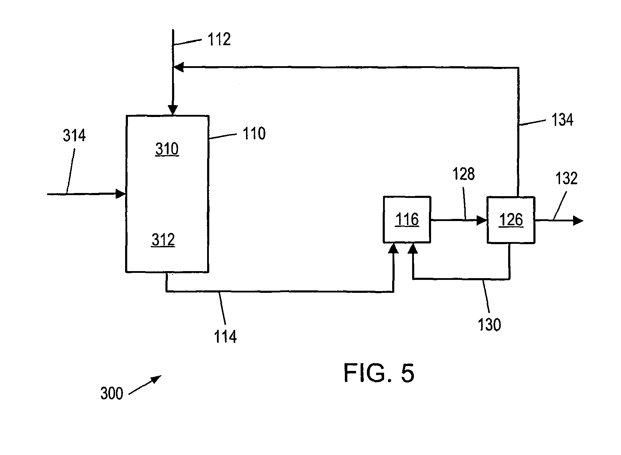

In an embodiment, a catalyst in dehydrogenation-isomerization unit 110 may be used in a stacked bed configuration. A stacked bed configuration may allow for the use of one or more catalysts in the reactor. A catalyst for dehydrogenation of paraffins and a catalyst for isomerization of olefins may enhance the selectivity of the catalysts and/or the process. A stacked bed configuration of dehydrogenation-isomerization unit 110 is, depicted for System 300 in FIG. 5. Operating conditions of the stacked bed configuration may be the same as for two-zone system described above for System 200. The first hydrocarbon stream may enter the dehydrogenation zone 310 via first conduit 112.

The dehydrogenation catalyst used in the stacked bed configuration may have nonacidic properties. The term "nonacidic," as used herein, refers to a catalyst that exhibits little skeletal isomerization activity. The dehydrogenation catalyst may include a noble metal, a Group IVA component, an alkali or alkaline earth component, a halogen component and/or a porous carrier material.

In certain embodiments, a noble metal may be dispersed throughout the dehydrogenation catalyst, An amount of noble metal may range between 0.01 weight percent to 5 weight percent, calculated on an elemental basis, of the final dehydrogenation catalyst. In certain embodiments, a dehydrogenation catalyst includes 0.1 weight percent to 1 weight percent platinum. The noble metal may be incorporated into the catalytic composite by techniques known in the art (e.g., co-precipitation, co-gelation, ion exchange, impregnation, deposition from a vapor phase or from an atomic source) before incorporation of other catalytic components. In some embodiments, a noble metal may be incorporated into the catalytic composite during incorporation of other catalytic components. In other embodiments, a noble metal may be incorporated into the catalytic composite after incorporation of other catalytic components. In certain embodiments, a noble metal may be incorporated by impregnation of the carrier material with a solution or suspension of a decomposable compound of the noble metal. For example, platinum may be added to a catalytic support by commingling the platinum with an aqueous solution of chloroplatinic acid. In other embodiments, optional components (e,g., nitric acid) may be added to the impregnating solution to assist in dispersing or fixing the noble metal in the final catalyst composite.

The Group IVA component, may include germanium, tin, lead or combinations thereof. In some embodiments, a Group IVA component may exist within the catalyst in an oxidation state above that of the noble metal. The Group IVA component may be present as an oxide. In certain embodiments, a Group IVA component may be combined with a carrier material. In some embodiments, a Group IVA component may be combined with the other catalytic components. In other embodiments, a Group IVA component may be dispersed throughout the catalyst. A Group IVA component may range between 0.01 weight percent to 5 weight percent, calculated on an elemental basis, of the final catalyst composite. In some embodiments, a catalyst includes 0.2 weight percent to 2 weight percent tin.

The Group IVA component may be incorporated in the catalytic composite according to generally known methods (e.g., co-precipitation, co-gelation, ion exchange and impregnation) before other catalytic components are incorporated. In some embodiments, a Group IVA component may be incorporated during incorporation of other catalytic components. In other embodiments, a Group IVA component may be incorporated after other catalytic components are incorporated. In some embodiments, a tin component may be incorporated by co-gelation with the porous carrier material. The tin may be incorporated in an alumina carrier material by mixing a soluble tin compound (e.g., stannous or stannic chloride) with an alumina hydrosol. A gelling agent (e.g., hexamethylenetetraamine) may be added to the tin-alumina hydrosol mixture, The tin-alumina hydrosol mixture may be dropped into an oil bath to form spheres containing alumina and tin. In other embodiments, a germanium component may be impregnated into a carrier material with a solution or suspension of a decomposable compound of germanium (e.g., germanium tetrachloride dissolved in an alcohol). In other embodiments, a lead component may be impregnated from a solution of lead nitrate in water.

In certain embodiments, an alkali or alkaline earth component may be included in the dehydrogenation catalyst. Alkali and alkaline earth component may include, but is not limited to, cesium, rubidium, potassium, sodium, lithium, barium, strontium, calcium and magnesium or mixtures thereof. The alkali or alkaline earth component may exist in the final catalytic composite in an oxidation state above that of the noble metal. The alkali or alkaline earth component maybe present as an oxide. In some embodiments, an alkali or alkaline earth metal may be combined with the carrier material. In certain embodiments an alkali or alkaline earth metal may be combined with other dehydrogenation catalytic components.

In other embodiments, an alkali or alkaline earth component may be dispersed throughout the catalytic composite. An amount of alkali or alkaline earth component may range from 0.01 weight percent to 15 weight percent, calculated on an elemental basis, of the final catalytic composite. In other embodiments, a dehydrogenation catalyst includes 1 weight percent to 3 weight percent potassium. In certain embodiments, an atomic ratio of the alkali or alkaline earth component to the noble metal may be greater than at least 10.

The alkali or alkaline earth component may be incorporated in the catalytic composite according to generally known methods (e.g., co-precipitation, co-gelation, ion exchange or impregnation) before other catalytic components are incorporated. In some embodiments, an alkali or alkaline earth component may be incorporated during incorporation of other catalytic components. In other embodiments, an alkali or alkaline earth component may be incorporated after other catalytic components are incorporated. For example, a potassium component may be impregnated into the carrier material with a solution of potassium nitrate. An atomic ratio of alkali or alkaline earth component to noble metal may be at least 10. In certain embodiments, an atomic ratio of the alkali or alkaline earth component to the noble metal component may range from 15 to 25.

A porous carrier material used in a dehydrogenation catalyst may include a porous, absorptive support with high surface area from 25 m²/g to 500 m²/g. The porous carrier material may have a melting point greater than the conditions utilized in the dehydrogenation zone. Examples of carrier materials include, but are not limited to, activated carbon, coke, charcoal, silica, silica gel, silicon carbide, synthetically prepared and/or naturally occurring clays and silicates, refractory inorganic oxides, crystalline zeolitic aluminosilicates, naturally occurring or synthetically prepared mordenite and/or faujasite, spinels or combinations of thereof. In certain embodiments, a carrier material may be gamma- or eta-alumina. In some embodiments, clays and silicates may or may not be acid treated (e.g., attapulgite, china clay, diatomaceous earth, fuller's earth, kaolin, kieselguhr, ceramics, porcelain, crushed firebrick, bauxite). Examples of refractory inorganic oxides include alumina, titanium dioxide, zirconium dioxide, chromium oxide, beryllium oxide, vanadium oxide, cerium oxide, hafnium oxide, zinc oxide, magnesia, boria, thoria, silica-alumina, silica-magnesia, chromia-alumina, alumina-boria, and silica-zirconia. Zeolitic aluminosilcates may be, in some embodiments, in the hydrogen form. In other embodiments, zeolitic aluminosilcates may be in a form that may be exchanged with metal cations. Examples of spinels include, but are not limited to, MgAl₂O₄, FeAl₂O₄, ZnAl₂O₄, CaAl₂O₄, and other like compounds having the formula MO-Al₂O₃ in which M is a metal having a valence of 2.

In certain embodiments, an alumina carrier material used in a dehydrogenation catalyst may be prepared in any suitable manner from synthetic or naturally occurring raw materials. The alumina carrier may be formed in any desired shape (e.g., spheres, pills, calces, extrudates, powders, granules). The alumina carrier may be utilized in any particle size. In certain embodiments, a sphere shape may be utilized. The particle may be 1/16 inch in diameter. In certain embodiments, a particle diameter of less than 1/32 inch may be utilized.

Alumina spheres may be prepared, in some embodiments, by converting aluminum metal into an alumina sol. An alumina sol may be prepared by reacting aluminum metal with a suitable peptizing acid and water. The resulting alumina sol and a gelling agent may be dropped into an oil bath to form spherical particles of an alumina gel. The resulting alumina gel may be converted to gamma- or eta-alumina carrier material using known techniques (e,g., by aging, drying and calcining).

In other embodiments, alumina cylinders may be prepared by mulling alumina powder with water and a suitable peptizing agent (e.g., nitric acid) to form an extrudable composition. The composition may be extruded through a suitably sized die then cut to form extrudate particles. Other shapes of the alumina carrier material may be prepared by conventional methods. After the alumina particles are shaped, they may be dried and calcined. The alumina carrier may be subjected to intermediate treatments (e.g., washing with water or a solution of ammonium hydroxide) during preparation.

The dehydrogenation catalyst may include a halogen component. The halogen component may include, but is not limited to, fluorine, chlorine, bromine, iodine or mixtures thereof. The halogen component may be present in a combined state with the porous carrier material. In certain embodiments, a halogen component may be dispersed throughout the catalytic composite. A halogen component may range from at least 0.2 weight percent to 15 weight percent, calculated on an elemental basis, of the final catalytic composite. In certain embodiments, a dehydrogenation catalyst contains 1 weight percent to 3 weight percent chlorine.

In certain embodiments, a catalyst composition may include at least 0.2 weight percent, calculated on an elemental basis, of a halogen component. The halogen component in the catalyst may improve the activity of the catalyst for dehydrogenating hydrocarbons. In some embodiments, an active halogen component may suppress carbon formation on the catalyst during the dehydrogenation process. An advantage of the catalyst composition may be that undesirable isomerization or cracking side reactions may be inhibited. In certain embodiments, halogen content may increase the acidity of the catalyst, The acidity may be lowered by steaming the dehydrogenation catalyst to remove excess halogen from the dehydrogenation catalyst.

A halogen component may be incorporated in the catalytic composite in any suitable manner. The incorporation of the halogen may be before preparation of the carrier material. In some embodiments, incorporation of a halogen may be during incorporation of other catalytic components. In other embodiments, incorporation of a halogen may be after other catalytic components are incorporated. In certain embodiments, an alumina sol carrier may contain a halogen, which may contribute to at least some portion of the halogen content in the final catalyst composite. In some embodiments, a halogen component, or a portion thereof, may be added to the catalyst composite during the incorporation of the carrier material with other catalyst components (e.g., using chloroplatinic acid to impregnate the platinum component). In other embodiments, a halogen component or a portion thereof may be added to the catalyst composite by contacting the catalyst with the halogen. In some embodiments, a halogen may be added to the catalyst as a compound, solution, suspension or dispersion containing the halogen, (e.g., hydrochloric acid) before or after other catalyst components are incorporated with the carrier material. In certain embodiments, a halogen component or a portion thereof may be incorporated by contacting the catalyst with a compound, solution, suspension or dispersion containing the halogen in a subsequent catalyst regeneration step. In the regeneration step, carbon deposited on the catalyst as coke during use of the catalyst in a hydrocarbon conversion process may be burned off the catalyst. The noble metal on the catalyst may be redistributed to provide a regenerated catalyst with performance characteristics similar to those of the fresh catalyst. The halogen component may be added during the carbon burn step or during the noble metal redistribution step (e.g., contacting the catalyst with a hydrogen chloride gas). In some embodiments, a halogen component may be added to the catalyst composite by adding the halogen or a compound, solution, suspension or dispersion containing the halogen (e.g., propylene dichloride) to the hydrocarbon feed stream. In other embodiments, a halogen component may be added to the recycle gas during operation of the dehydrogenation unit.

In some embodiments, a dehydrogenation catalyst may include a sulfur component ranging from 0.01 weight percent to 10 weight percent, calculated on an elemental basis, of the final catalytic composition. The sulfur component may be incorporated into the catalytic composite in any suitable manner. In certain embodiments, sulfur or a compound containing sulfur (e.g., hydrogen sulfide or a lower molecular weight mercaptan) maybe contacted with the catalyst composition in the presence of hydrogen at a temperature ranging from 10 °C to 540 °C under anhydrous conditions. A hydrogen to sulfur ratio, in some embodiments, may be 100.

The dehydrogenation catalyst, in some embodiments, may also contain other, additional components or mixtures thereof, which act alone or in concert, as catalyst modifiers to improve catalyst activity, selectivity or stability. Examples of catalyst modifiers include, but are not limited to, antimony, arsenic, beryllium, bismuth, cadmium, calcium, chromium, cobalt, copper, gallium, gold, indium, iron, lithium, manganese, molybdenum, nickel, rhenium, scandium, silver, tantalum, thallium, titanium, tungsten, uranium, zinc and zirconium. Catalytic modifiers may be added in any suitable manner to the carrier material during preparation of the dehydrogenation catalyst. In other embodiments, catalytic modifiers may be added in any suitable manner after preparation of the dehydrogenation catalyst. In some embodiments, catalytic modifiers may be added in any suitable manner to the catalytic composite before other catalytic components are incorporated. In certain embodiments, catalytic modifiers may be added during incorporation of other catalytic components. In other embodiments, catalytic modifiers may be added after other catalytic components are incorporated. A description of a dehydrogenation catalyst may be found in U.S. Patent No. 4,506,032 to Imai et al., entitled "Dehydrogenation Catalyst Composition."

The olefinic hydrocarbon stream may pass into isomerization zone 312. In certain embodiments, a temperature decrease from dehydrogenation zone 310 to isomerization zone 312 may be necessary to prevent cracking of the olefinic hydrocarbon stream as it enters the isomerization zone. Cool hydrogen gas may be introduced to dehydrogenation zone 310 via gas conduit 314 to control temperatures in dehydrogenation zone 310. In isomerization zone 312, at least a portion of the olefins in the olefinic hydrocarbon stream may be isomerized to branched olefins to produce a second hydrocarbon stream.

In certain embodiments, an isomerization catalyst may be the same as described for isomerization of olefins in System 200. A description of the isomerization catalyst may be found in U.S. Patent No. 5,510,306 to Murray, entitled "Process For Isomerizing Linear Olefins to Isoolefins." In some embodiments, 0.01 weight percent to 5 weight percent of a noble metal may be added to an isomerization catalyst used in a stacked bed configuration to increase the dehydrogenation activity of the zeolitic catalyst. Common metal incorporation methods (e.g., impregnation, noble metal ion exchange, co-mulling) may be used to incorporate a noble metal (e.g., platinum, palladium) into a zeolite to produce a working catalyst useful in the dehydrogenation-isomerization of paraffins.

The second hydrocarbon stream may exit isomerization zone 312 and enter hydroformylation unit 116 via second conduit 114. At least a portion of the olefins in the second hydrocarbon stream may be hydroformylated to produce a hydroformylation reaction stream as described for System 100. At least a portion of the hydroformylation reaction stream may be separated into a bottoms stream and a top stream using generally known methods. The top stream may be purified and separated as described for System 100 to produce a paraffins and unreacted olefins stream and a crude aliphatic alcohol product stream. The crude aliphatic alcohol product stream may be further purified as described for System 100 to produce an aliphatic, alcohol product stream. The hydroformylation reaction mixture stream may enter separator 126 via third conduit 128. In separator 126 at least two streams, a bottom stream and top stream, may be produced as previously described for System 100. The bottom stream may be recycled to hydroformylation unit 116 via bottom stream recycle conduit 130. The top stream may be purified and separated into at least two streams, a paraffins and unreacted olefins stream and a crude aliphatic alcohol product stream. At least a portion of the paraffins and unreacted olefins stream may be recycled, combined with other process streams, sent to other processing units and/or sent to a storage vessel. The crude aliphatic alcohol product stream may be further purified as described for System 100 to produce an aliphatic alcohol product stream. The aliphatic alcohol product stream may include branched aliphatic alcohols (e.g., branched primary aliphatic alcohols). The aliphatic alcohol product stream may be transported via product conduit 132 to be stored on site, sold commercially, transported off-site and/or utilized in other processing units.

At least a portion of the paraffins and unreacted olefins stream may be combined with the first hydrocarbon stream in first conduit 112 to produce a combined hydrocarbon stream via fourth conduit 134. The combined hydrocarbon stream may enter dehydrogenation zone 310 of dehydrogenation-isomerization unit 110 via first conduit 112. The combined hydrocarbon stream entering dehydrogenation zone 310 continues the dehydrogenation-isomerization process and hydroformylation process to produce aliphatic alcohols. By recycling the paraffins and unreacted olefins stream, the yield of product may be maximized. In an embodiment, a paraffins and unreacted olefins stream may directly enter dehydrogenation-isomerization unit 110 through one or more entry points.

In some embodiments, an olefin and paraffin concentration in hydroformylation unit 116 may be adjusted depending on the source of the olefin stream entering the hydroformylation unit. At least a portion of a third hydrocarbon stream may be introduced into the second conduit upstream of the hydroformylation unit as previously described for System 100. The combined stream may be introduced into hydroformylation unit 116 via second conduit 114. At least a portion of the cleans in the combined stream may be hydroformylated to produce aliphatic alcohols.

In an embodiment, a third hydrocarbon stream includes a paraffin content between 50 percent and 99 percent relative to the total hydrocarbon content. In certain embodiments, a paraffin content of the third hydrocarbon stream ranges between 60 percent and 90 percent relative to the total hydrocarbon content. In other embodiments, a paraffin content of the third hydrocarbon stream may be greater than 80 percent relative to the total hydrocarbon content.

In an embodiment, an olefin content of a third hydrocarbon stream ranges between 1 percent and 99 percent relative to the total hydrocarbon content. In other embodiments, an olefin content of a third hydrocarbon stream may be greater than 80 percent relative to the total hydrocarbon stream.

Aliphatic alcohols may be converted to oxy alcohols, sulfates or other commercial products. At least a portion of the aliphatic alcohols in the alcohol product stream may be reacted in an oxyalkylation unit with an epoxide (e.g., ethylene oxide, propylene oxide, butylene oxide) in the presence of a base to produce an oxyalkyl alcohol. Condensation of an alcohol with an epoxide allows the alcohol functionality to be expanded by one or more oxy groups. The number of oxy groups may range from 3 to 12. For example, reaction of an alcohol with ethylene oxide may produce alcohol products having between 3 to 12 ethoxy groups. Reaction of an alcohol with ethylene oxide and propylene oxide may produce alcohols with an ethoxy/propoxy ratio of ethoxy to propoxy groups from 4:1 to 12:1. In some embodiments, a substantial proportion of alcohol moieties may become combined with more than three ethylene oxide moieties. In other embodiments, an approximately equal proportion may be combined with less than three ethylene oxide moieties. In a typical oxyalkylation product mixture, a minor proportion of unreacted alcohol may be present in the product mixture. In an embodiment, at least a portion of the aliphatic alcohol product stream may be formed by condensing a C₅ to C₃₁ aliphatic alcohol with an epoxide. In certain embodiments, a C₅ to C₁₅ branched primary alcohol may be condensed with ethylene oxide and/or propylene oxide. In other embodiments, a C₁ to C₁₇ branched primary alcohol may be condensed with ethylene oxide and/or propylene oxide. The resulting oxyalkyl alcohols may be sold commercially, transported off-site, stored on site and/or used in other processing units. In some embodiments, an oxyalkyl alcohol may be sulfated to from an anionic surfactant.

In an embodiment, at least a portion of the alcohols in the aliphatic alcohol product stream may be added to a base. The base may be an alkali metal or alkaline earth metal hydroxide (e.g., sodium hydroxide or potassium hydroxide). The base may act as a catalyst for the oxyalkylation reaction. An amount from 0.1 percent by weight to 0.6 percent by weight of a base, based on the total weight of alcohol, may be used for oxyalkylation of an alcohol. In an embodiment, a weight percent of a base may range from 0.1 percent by weight to 0.4 percent by weight based on the total alcohol amount. The reaction of the alcohol with the base may result in formation of an alkoxide. The resulting alkoxide may be dried to remove any water present. The dried alkoxide may be reacted with an epoxide. An amount of epoxide used may be from 1 mole to 12 moles of epoxide per mole of alkoxide. A resulting alkoxide-epoxide mixture may be allowed to react until the epoxide is consumed. A decrease in overall reaction pressure may indicate that the reaction is complete.

Reaction temperatures in an oxyalkylation unit may range from 120 °C to 220 °C. In an embodiment, reaction temperatures may range from 140 °C to 160 °C. Reaction pressures may be achieved by introducing to the reaction vessel the required amount of epoxide. Epoxides have a high vapor pressure at the desired reaction temperature. For consideration of process safety, the partial pressure of the epoxide reactant may be limited, for example, to less than 4 atmospheres (413 kPa). Other safety measures may include diluting the reactant with an inert gas such as nitrogen. For example, inert gas dilution may result in a vapor phase concentration of reactant of 50 percent or less. In some embodiments, an alcohol-epoxide reaction may be safely accomplished at a greater epoxide concentration, a greater total pressure and a greater partial pressure of epoxide if suitable, generally known, safety precautions are taken to manage the risks of explosion. With respect to ethylene oxide; a total pressure, from 3 atmospheres (304 kPa) to 7 atmospheres (709kPa) may be used. Total pressures of ethylene oxide from 1 atmosphere (101 kPa) to 4 atmospheres (415 kPa) may be used in certain embodiments: In an embodiment, total pressures from 1.5 atmospheres (150 kPa) to 3 atmospheres (304 kPa) with respect to ethylene oxide may be used. The pressure may serve as a measure of the degree of the reaction. The reaction may be considered substantially complete when the pressure no longer decreases with time.

Aliphatic alcohols and oxyalkyl alcohols may be derivatized, to form compositions (e.g., sulfonates, sulfates, phosphates) useful in commercial product formulations (e,g., detergents, surfactants, oil additives, lubricating oil formulations). For example, alcohols may be sulfurized with SO₃ to produce sulfates. The term "sulfurized" refers to a sulfur atom or sulfur containing functionality being added to a carbon or oxygen. Sulfurization processes are described in U.S. Patent No. 6,462,215 to Jacobson et al., entitled "Sulfonation, Sulfation and Sulfamation"; U.S. Patent No. 6,448,435 to Jacobson et al., entitled "Sulfonation, Sulfation and Sulfamation"; U.S. Patent No. 3,462,525 to Levinsky et al, entitled, "Dental Compositions Comprising Long-Chain Olefin Sulfonates;" U.S. Pat. No. 3,428,654 to Rubinfeld et al., entitled, "Alkene Sulfonation Process and Products;" U.S. Patent No. 3,420,875 to DiSalvo et al., entitled, "Olefin Sulfonates;" U.S. Patent No. 3,506,580 to Rubinfeld et al., entitled, "Heat-Treatment Of Sulfonated Olefin Products;" and U.S. Patent No. 3,579,537 to Rubinfeld, entitled, Process For Separation Of Sultones From Alkenyl Sulfonic Acids."

A general class of aliphatic alcohol sulfates may be characterised by the chemical formula: (R-O-(A)*ₓ*-SO₃)*ₙ*M R' represents the aliphatic moiety. "A" represents a moiety of an alkylene oxide; *x* represents the average number of A moieties per R-O moiety and may range from 0 to 15; and *n* is a number depending on the valence of cation M. Examples of cation M include, but are not limited to, alkali metal ions, alkaline earth metal ions, ammonium ions and/or mixtures thereof. Examples of cations include, but are not limited to, magnesium, potassium, monoethanol amine, diethanol amine or triethanol amine.

Aliphatic and oxyalkyl alcohols may be sulfated in a sulfation unit Sulfation procedures may include the reaction of sulfur trioxide (SO₃), chlorosulfonic acid (ClSO₃H), sulfamic acid (NH₂SO₃H) or sulfuric acid with an alcohol. In an embodiment, sulfur trioxide in concentrated (e.g., fuming) sulfuric acid may be used to sulfate alcohols. The concentrated sulfuric acid may have a concentration of 75 percent by weight to 100 percent by weight in water. In an embodiment, concentrated sulfuric acid may have a concentration of 85 percent by weight to 98 percent by weight in water. The amount of sulfur trioxide may range from 0.3 mole to 1.3 moles of sulfur trioxide per mole of alcohol In certain embodiments, an amount of sulfur trioxide may range from 0.4 moles to 1.0 moles of sulfur trioxide per mole of alcohol.

In an embodiment, a sulfur trioxide sulfation procedure may include contacting a liquid alcohol or an oxyalkyl alcohol and gaseous sulfur trioxide in a falling film sulfator to produce a sulfuric acid ester of the alcohol. The reaction zone of the falling film sulfator may be operated at about atmospheric pressure and at a temperature in the range from 25 °C to 70 °C. The sulfuric acid ester of the alcohol may exit the falling film sulfator and enter a neutralization reactor. The sulfuric acid ester may be neutralized with an alkali metal solution to form the alkyl sulfate salt or the oxyalkyl sulfate salt. Examples of an alkali metal solution may include solutions of sodium or potassium hydroxide.

The derivatized alcohols may be used in a wide variety of applications. An example of an application includes detergent formulations. Detergent formulations include, but are not limited to, granular laundry detergent formulation, liquid laundry detergent formulations, liquid dishwashing detergent formulations and miscellaneous formulations. Examples of miscellaneous formulations may include general purpose cleaning agents, liquid soaps, shampoos and liquid scouring agents.

Granular laundry detergent formulations may include a number of components besides the derivatized alcohols (e.g., surfactants, builders, co-builders, bleaching agents, bleaching agent activators, foam controlling agents, enzymes, anti-graying agents, optical brighteners and stabilizers). Examples of other surfactants may include ionic, nonionic, amphoteric or cationic surfactants.

Liquid laundry detergent formulations may include the same components as granular laundry detergent formulations. In certain embodiments, liquid laundry detergent formulations may include less of an inorganic builder component than granular laundry detergent formulations. Hydrotropes may be present in the liquid detergent formulations. General purpose cleaning agents may include other surfactants, builders, foam control agents, hydrotropes and solubilizer alcohols.

Formulations may typically include one or more inert components. For example, the balance of liquid detergent formulations may typically be an inert solvent or diluent (e.g., water). Powdered or granular detergent formulations typically contain quantities of inert filler or carrier materials.

### EXAMPLES

Example 1. Isomerization of Olefins in a Fischer-Tropsch derived Hydrocarbon Stream: Carbon monoxide and hydrogen were reacted under Fischer-Tropsch process conditions to yield a hydrocarbon mixture of linear paraffins, linear olefins, a minor amount of dienes and a minor amount of oxygenates. The Fischer-Tropsch hydrocarbon stream was separated into different hydrocarbon streams using fractional distillation techniques. A hydrocarbon stream containing olefins and paraffins with an average number of carbon atoms from 8 to 10 was obtained. The composition of the resulting C₈-C₁₀ hydrocarbon stream was analysed by gas chromatography and is tabulated in Table 1.

**Table 1**

| **Fischer-Tropsch Hydrocarbon Stream Composition** | **Wt.%** |
|---|---|
| C₇ and lighter hydrocarbons | 0.12 |
| C₈ branched olefins | 0.02 |
| C₈ linear olefins | 0.75 |
| 1-Octene | 0.69 |
| n-Octane | 2.21 |
| C₉ branched olefins | 0.16 |
| C₉ linear olefins | 8.52 |
| 1-Nonene | 8.07 |
| n-Nonane | 20.03 |
| C₁₀ branched olefins | 0.28 |
| C₁₀ linear olefins | 22.92 |
| 1-Decene | 20.87 |
| n-Decane | 41.12 |
| C₁₁ and heavier hydrocarbons | 0.21 |
| C₉-C₁₁ alcohols | 3.56 |

A zeolite catalyst used for isomerization of linear olefins in the hydrocarbon stream was prepared in the following manner. Ammonium-ferrierite (645 grams) exhibiting a 5.4% loss on ignition and exhibiting the following properties: molar silica to alumina ratio of 62:1, surface area of 369 square meters per gram (P/Po=0.03), soda content of 480 ppm and n-hexane sorption capacity of 7.3 g per 100 g of ammonium-ferrierite was loaded into a Lancaster mix muller. CATAPAL® D alumina (91 grams) exhibiting a loss on ignition of 25.7% was added to the muller. During a five-minute mulling period, 152 milliliters of deionized water was added to the alumina/ammonium-ferrierite mixture. Next, a mixture of 6.8 grams glacial acetic acid, 7.0 grams of citric acid and 152 milliliters of deionized water was slowly added to the alumina/ammonium-ferrierite mixture in the muller to peptize the alumina. The resulting alumina/ammonium-ferrierite/acid mixture was mulled for 10 minutes. Over a period of 15 minutes, a mixture of 0.20 grams of tetraamine palladium nitrate in 153 grams of deionized water was slowly added to mulled alumina/ammonium-ferrierite/acid mixture. The resulting mixture exhibited a 90:10 ratio of zeolite to alumina and a loss on ignition of 43.5%. The zeolite/alumina mixture was shaped by extruding the mixture through a stainless steel die plate (1/16" holes) of a 2.25 inch Bonnot extruder.
The moist zeolite/alumina extrudate was dried at 125°C for 16 hours. After drying, the zeolite/alumina extrudate was longsbroken manually. The zeolite/alumina extrudate was calcined in flowing air at 200°C for two hours. The temperature was raised to a maximum temperature of 500°C and the zeolite/alumina extrudate was calcined for an additional two hours to yield an isomerization catalyst. The isomerization catalyst was allowed to cool in a dessicator under a nitrogen atmosphere.
Stainless steel tubing, 1 inch OD, 0.6 inch ID and 26 inches long, was used as an isomerization reactor. A thermowell extended 20 inches from the top of the stainless steel reactor tube. To load the reactor tube, the reactor tube was inverted and a piece of glass wool was transferred down the wall of the reactor tube, over the thermowell and positioned at the bottom of the reactor tube to serve as a plug for the reactor tube. Silicon carbide (20 mesh) was added to a depth of about 6 inches to the reactor tube. A second piece of glass wool was placed over the silicon carbide. A mixture of 6.0 grams of the isomerization catalyst particles (6-20 mesh) and 45 grams of fresh silicon carbide (60-80 mesh) was added to the reactor tube in two parts. The two-part addition distributed the isomerization catalyst evenly in the reactor tube and resulted in an isomerization catalyst bed of about 10 inches in length. A third piece of glass wool was added to the top of the catalyst in the reactor tube. Silicon carbide (20 mesh) was layered onto the third piece of glass wool. A fourth piece of glass wool was positioned over the silicon carbide to serve as a plug for the bottom of the reactor tube. To monitor the temperature of the reaction at various points in the reactor tube, a multipoint thermocouple was inserted into the thermowell of the reactor tube. The temperature above, below and at three different places in the catalyst bed was monitored. The reactor tube was inverted and installed in the furnace. The reactor tube was heated to the operating temperature of 280 °C over a four-hour period under flowing nitrogen. Once the temperature of 280 °C was obtained, the reactor tube was held at the operating temperature for an additional two hours to condition the isomerization catalyst.
After conditioning the isomerization catalyst, the hydrocarbon stream was pumped through the reactor tube at a flow rate of 60 g/hr. Nitrogen, at a flow rate of 6 L/hr, was passed over the isomerization catalyst simultaneously with the hydrocarbon stream. The hydrocarbon stream was vaporized before contacting the isomerization catalyst. The reactor tube was operated at an outlet pressure of 20 kPa above atmospheric pressure.
In Table 2, the weight percent of C₈-C₁₀ branched olefins, C₈-C₁₀ linear olefins and C₈-C₁₀ paraffins in the hydrocarbon stream at 0 hours and in the reactor tube effluent after 24 and 48 hours of isomerization is tabulated. Greater than 90% of the linear olefins in the hydrocarbon stream were converted into branched olefins in the isomerization reactor. During the isomerization step, a small amount of material boiling below C₈ was generated from cracking side reactions. In addition, a portion of the C₉-C₁₁ alcohols present in the feed was dehydrated to yield additional olefins in the product. The average number of alkyl branches on the C₈-C₁₀ olefins in the product was found to be 1.0 as determined by ¹H NMR analysis.

**Table 2**

| **Fischer-Tropsch Hydrocarbon Stream Composition During Isomerization Reaction** | **0 Hr** | **24 Hr** | **48 Hr** |
|---|---|---|---|
| | **Wt.%** | **Wt.%** | **Wt.%** |
| C₈-C₁₀ branched olefins | 0.46 | 33.04 | 33.16 |
| C₈-C₁₀ linear olefins | 32.19 | 2.52 | 2.54 |
| C₈-C₁₀ paraffins | 63.19 | 63.32 | 63.27 |
| Branched to linear C₈₋₁₀ olefin ratio | 0.1 | 13.1 | 13.1 |

Example 2. Isomerization of 1-Dodecene: 1-dodecene was obtained from Shell Chemical Co. The composition of 1-dodecene, as assayed by gas chromatography, is tabulated in Table 3.

**Table 3**

| **1-Dodecene Composition** | **Wt.%** |
|---|---|
| 1-Dodecene | 98.0 |
| Other C₁₀-C₁₄ olefins | 1.2 |
| <C₁₀ hydrocarbons | 0.2 |
| >C₁₄ hydrocarbons | 0.2 |
| Paraffins | 0.4 |
| Total C₁₀-C₁₄ hydrocarbons | 99.6 |

1-dodecene was isomerized using the same reactor tube design and isomerization catalyst preparation as described in Example 1. A stream of 1-dodecene was pumped through a reactor tube at a flow rate of 90 g/hr. Nitrogen, at a flow rate of 6 L/hr, was passed over the isomerization catalyst simultaneously with the stream of 1-dodecene. The stream of 1-dodecene was vaporised before contacting the isomerization catalyst. The reactor tube was operated at an outlet pressure of 20 kPa above atmospheric pressure and a temperature of 290°C.
Table 4 is a tabulation of the weight percent of less than C₁₀, C₁₀-C₁₄ and greater than C₁₄ molecules in 1-dodecene at 0 hours and the reactor tube effluent after 168 and 849 hours. Linear C₁₀-C₁₄ olefins were converted in a 94% yield to branched C₁₀-C₁₄ olefins after a 168 hr processing time. During the isomerization step, less than 3 weight percent of material boiling below C₁₀ was generated from cracking side reactions. The average number of alkyl branches on the C₁₀-C₁₄ olefins in the product was determined to be 1.3 by ¹H NMR analysis.

**Table 4**

| **1-Dodecene Stream Composition During Isomerization Reaction** | **0 Hr** | **168 Hr** | **849 Hr** |
|---|---|---|---|
| | **Wt.%** | **Wt.%** | **Wt.%** |
| <C₁₀ hydrocarbons | 0.2 | 2.5 | 2.4 |
| C₁₀-C₁₄ hydrocarbons | 99.6 | 97.2 | 97.4 |
| >C₁₄ hydrocarbons | 0.2 | 0.3 | 0.2 |
| Branched C₁₀-C₁₄ olefins | 0.6 | 93.2 | 93.4 |
| Linear C₁₀-C₁₄ olefins | 99.0 | 2.8 | 2.9 |
| Paraffins | 1.0 | 2.0 | 1.9 |

Example 3. Dehydrogenation of Dodecane with Minimal Isomerization: Dodecane was obtained from Aldrich Chemical Company and stored under nitrogen before being processed. The composition of dodecane, as assayed by gas chromatography, is tabulated in Table 5.

**Table 5**

| **Dodecane Composition** | **Wt.%** |
|---|---|
| Dodecane | 99.3 |
| <C₁₀ hydrocarbons | <0.1 |
| C₁₀, C₁₁, C₁₃ and C₁₄ hydrocarbons | <0.6 |
| >C₁₄ hydrocarbons | <0.1 |
| Other C₁₀-C₁₄ olefins | <0.1 |

A paraffin dehydrogenation catalyst was prepared according to Example 1 (catalyst A) of U.S. Patent No. 4,430,517 to Imai et al., entitled "Dehydrogenation Process Using A Catalytic Composition." The resulting catalyst included 0.8 wt.% platinum, 0.5 wt.% tin, 2.7 wt.% tin, 2.7 wt.% potassium and 1.3 wt.% chlorine on a gamma-alumina support. The atomic ratio of potassium to platinum for this catalyst was 16.8.
The dehydrogenation catalyst was prepared by dissolving substantially pure aluminum pellets in a hydrochloric acid solution. An amount of stannic chloride was added to the resulting solution to provide a final composite containing 0.5 weight % tin and stirred to distribute the tin component evenly throughout the mixture. Hexamethylenetetramine was added to the resulting tin mixture and the resulting tin-amine mixture was dropped into an oil bath in a manner to form spherical particles having an average particle diameter of about 1/16 inch. The spheres were aged, washed with an ammoniacal solution, dried and calcined to form a spherical gamma-alumina carrier material. The resulting spheres contained about 0.5 weight % tin in the form of tin oxide. More details about the method of preparing the alumina carrier material are disclosed in U.S. Patent No. 2,620,314 to Hoesktra, entitled, "Spheroidal Alumina."
The tin-alumina composite was contacted with a deionized solution of chloroplatinic acid and hydrochloric acid (2 weight percent based on alumina weight) in a rotary drier for 15 minutes at room temperature. The amount of chloroplatinic acid used was the amount necessary to incorporate 0.8 weight percent platinum into the tin-alumina composite. The solution was then heated and purged with nitrogen to remove water resulting in a platinum-chlorine-tin-alumina composite. The incorporated chlorine was removed by heating the platinum-chlorine-tin-alumina composite to 550 °C and treating the composite with a 50/50 air/80 °C steam mixture at a gas hourly space velocity (GHSV) of 300 hr⁻¹. After treatment with the air/steam mixture, the platinum-tin-alumina composite contained less than 0.1 weight percent chlorine.
The platinum-tin-alumina composite was contacted with a deionized water solution of potassium nitrate. The amount of potassium nitrate used was the amount necessary to incorporate 2.7 weight percent of potassium in the platinum-tin-alumina composite. The water was removed from the platinum-tin-potassium-alumina composite by heating the composite to 100 °C under a purge of dry air (1000 hr⁻¹ GHSV) for 0.5 hour. The temperature was raised to 525 °C and the platinum-tin-potassium alumina composite was treated with a stream of hydrochloric acid (12 cc/hr, 0.9 M HC1) and a stream of 50/50 air/80 °C steam mixture (300 hr⁻¹ GHSV) to incorporate chlorine into the platinum-tin-potassium-alumina composite. The platinum-tin-potassium-chlorine-alumina composite was dried at 525 °C under a purge of dry air (1000 hr⁻¹ GHSV). The resulting catalyst spheres had an average particle diameter of 1/16 inch and were crushed and sized into 6-20 mesh particle before testing.
Stainless steel tubing, 1 inch OD, 0.6 inch ID and 26 inches long, was used as an isomerization reactor. A thermowell extended 20 inches from the top of the stainless steel reactor tube. To load the reactor tube, the reactor tube was inverted and a piece of glass wool was transferred down the wall of the reactor tube, over the thermowell and positioned at the bottom of the reactor tube to serve as a plug for the reactor tube. Silicon carbide (20 mesh) was added to a depth of about 6 inches to the reactor tube. A second piece of glass wool was placed over the silicon carbide. A mixture of 6.0 grams of platinum-tin on alumina catalyst particles (6-20 mesh) and 45 grams of fresh silicon carbide (60-80 mesh) was added to the reactor tube in two parts. The two-part addition distributed the catalyst evenly in the reactor tube and resulted in a catalyst bed of about 10 inches in length. A third piece of glass wool was added to the top of the catalyst in the reactor tube. Silicon carbide (20 mesh) was layered onto the third piece of glass wool. A fourth piece of glass wool was positioned over the silicon carbide to serve as a plug for the bottom of the reactor tube. To monitor the temperature of the reaction at various points in the reactor tube, a multipoint thermocouple was inserted into the thermowell of the reactor tube. The temperature above, below and at three different places in the catalyst bed was monitored. The reactor tube was inverted and installed in the furnace. The reactor tube was purged with nitrogen. The reactor tube was heated to the operating temperature of 425°C over a four-hour period under flowing nitrogen (250 standard liters per hour). Once the temperature of 425°C was obtained, the reactor tube was held at the operating temperature for an additional two hours. The catalyst was presulfided by flowing a 1% mixture of hydrogen sulfide gas in hydrogen gas at 425 °C for five minutes through the reactor tube. After 5 minutes, the hydrogen sulfide in hydrogen gas flow was switched to a hydrogen gas flow through the reactor tube.
After presulfiding the catalyst, the reactor tube was maintained at 425 °C for eight hours. After eight hours, the reactor tube pressure was increase to 25 psig with hydrogen gas. Dodecane was pumped through the reactor tube at a flow rate of 40 g/hr at a hydrogen flow rate of 125 standard liters per hour. After four hours, the dodecane stream was increased to 80 g/hr. After obtaining a flow rate of 80 g/hr, the reactor tube temperature was raised to 460 °C. The reactor tube was sampled every eight hours after obtaining the operating temperature of 460°C.
After twenty-four hours the weight percent of dodecane was 11.4 weight percent as depicted in Table 6. At a temperature of 479 °C, the conversion of dodecane to olefins was 16 weight percent after twenty-four hours. Of the olefins, formed 84 weight percent were mono olefins, 4.1 weight percent were aromatic compounds and 7.5 weight percent were di-olefins. Of the total amount of olefins formed, 6 percent were branched, as determined by ¹H NMR analysis.

**Table 6**

| **Test Results.** | |
|---|---|
| Conversion (wt.%) after 24 hours on-stream at 460 °C. | 11.4 |
| Temperature required for 16 wt. % conversion | 479 °C |
| Selectivity to mono olefins at 16 wt. % conversion. | 84 wt.% |
| Selectivity to aromatics at 16 wt. % conversion. | 4.1 wt.% |
| Selectivity to di-olefins at 16 wt. % conversion. | 7.5 wt.% |
| % Branched C₁₂ olefins in total C₁₂ olefins | 6 |

Example 4. Dehydrogenation-Isomerization of Dodecane: Dodecane was obtained from Aldrich Chemical Company and stored under nitrogen before being processed. The composition of dodecane, as assayed by gas chromatography, is tabulated in Table 5.
A dehydrogenation-isomerization catalyst was prepared in the following manner. Ammonium-ferrierite (645 grams) exhibiting a 5.4% loss on ignition and exhibiting the following properties: molar silica to alumina ratio of 62:1, surface area of 369 square meters per gram (P/Po=0.03), soda content of 480 ppm and n-hexane sorption capacity of 7.3 g per 100 g of ammonium-ferrierite was loaded into a Lancaster mix muller. CATAPAL® D alumina (91 grams) exhibiting a loss on ignition of 25.7% was added to the muller. During a five-minute mulling period, 152 milliliters of deionized water was added to the alumina/ammonium-ferrierite mixture. Next, a mixture of 6.8 grams glacial acetic acid, 7.0 grams of citric acid and 152 milliliters of deionized water was slowly added to the alumina/ammonium-ferrierite mixture in the muller to peptize the alumina. The resulting alumina/ammonium-ferrierite/acid mixture was mulled for 10 minutes. Over a period of 15 minutes, a mixture of 0.20 grams of tetraamine palladium nitrate in 153 grams of deionized water was slowly added to mulled alumina/ammonium-ferrierite/acid mixture. The resulting mixture exhibited a 90:10 ratio of zeolite to alumina and a loss on ignition of 43.5%. The zeolite/alumina mixture was shaped by extruding the mixture through a stainless steel die plate (1/16" holes) of a 2.25 inch Bonnot extruder.
Six grams of the resulting zeolite/alumina mixture was impregnated with an aqueous solution of sodium hexachloroplatinate [IV] hexahydrate to incorporate 0.8 wt.% platinum into the 1/16 inch extrudate. The moist zeolite/alumina platinum impregnated extrudate was dried at 125°C for 2 hours in flowing air. The temperature was raised to a maximum temperature of 500°C and the zeolite/alumina platinum impregnated extrudate was calcined to yield a dehydrogenation-isomerization catalyst. The calcined catalyst was crushed and sized into 6-20 mesh particles before testing.
Dodecane was dehydrogenated and isomerized using the same reactor tube design as described in Example 3. A 16.1 weight percent conversion of dodecane to olefins was observed after twenty-fours hours at 459 °C. As tabulated in Table 7, of the olefins formed 86 weight percent were mono olefins, 1.2 weight percent were aromatic compounds and 6.8 weight percent were di-olefins. Of the total amount of olefins formed, 86 percent were branched, as determined by ¹H NMR analysis.

**Table 7**

| **Test Results.** | |
|---|---|
| Conversion (wt.%) after 24 hours on-stream at 460 °C. | 16.1 |
| Temperature required for 16 wt. % conversion | 459 °C |
| Selectivity to mono olefins at 16 wt. % conversion. | 86 wt.% |
| Selectivity to aromatics at 16 wt. % conversion. | 1.2 wt.% |
| Selectivity to di-olefins at 16 wt. % conversion. | 6.8 wt. % |
| % Branched C₁₂ olefins in total C₁₂ olefins | 86 |

Example 5. Dehydrogenation-Isomerization Catalyst: A zeolite portion of a dehydrogenation-isomerization catalyst was prepared as in Example 4. Six grams of the resulting zeolite/alumina mixture was impregnated with an aqueous solution of tetraamine palladium nitrate to incorporate 0.8 wt.% palladium into the 1/16 inch extrudates.
The moist zeolite/alumina palladium impregnated extrudate was dried at 125°C for 2 hours in flowing air. The temperature was raised to a maximum temperature of 500°C and the zeolite/alumina platinum impregnated extrudate was calcined to yield a dehydrogenation-isomerization catalyst. The calcined catalyst was carefully crushed and sized into 6-20 mesh particles before testing.
Example 6. Dehydrogenation-Isomerization Catalyst: A dehydrogenation-isomerization catalyst was prepared according to the method for catalyst D of U.S. Patent No. 5,648,585 to Murray et al., entitled, "Process For Isomerizing Linear Olefins To Isoolefins."
Ammonium-ferrierite having a molar silica to alumina ratio of 62:1, a surface area of 369 m2/g (P/Po=0.03), a soda content of 480 ppm wt and a n-hexane sorption capacity of 7.3 grams per 100 grams of zeolite was used. The catalyst components were mulled using a Lancaster mix muller. The mulled catalyst material was extruded using a Bonnot pin barrel extruder. The binder utilized was CATAPAL® D alumina from Sasol. METHOCEL ® F4M, hydroxypropyl methylcellulose, from The Dow Chemical Company was used as an extrusion aid.
The Lancaster mix muller was loaded with 632 grams of ammonium ferrierite (LOI of 3.4%) and 92 grams of CATAPAL®D alumina (LOI of 26.2%). The alumina was blended with the ferrierite for five minutes during which time 156 milliliters of de-ionized water was added. A mixture of 6.8 grams of glacial acetic acid and 156 milliliters of de-ionized water were added slowly to the muller in order to peptize the alumina. The mixture was mix-mulled for 10 minutes. Tetraamine platinum nitrate and Tetraamine palladium nitrate were added to the mix-muller in order to produce a catalyst that contained 0.25 wt. % palladium and 0.55 wt. % platinum. Ten grams of METHOCEL® F4M hydroxypropyl methylcellulose was added and the zeolite/alumina was mulled for 15 additional minutes. The extrudate was transferred to a Bonnot pin barrel extruder and extruded using a stainless steel die plate with 1/16 inch holes. The extrudate was dried at 120 °C for 16 hours and then calcined in air at 500 °C for 2 hours. The calcined catalyst was carefully crushed and sized into 6-20 mesh particles before testing.

## Claims

1. A method for the production of aliphatic alcohols, comprising:
introducing a first hydrocarbon stream comprising olefins and paraffins into a dehydrogenation-isomerization unit, wherein the dehydrogenation-isomerisation unit is configured to dehydrogenate at least a portion of the paraffins in the first hydrocarbon stream to olefins, and wherein the dehydrogenation-isomerization unit is further configured to isomerize at least a portion of linear olefins to branched olefins, and wherein at least a portion of the unreacted components of the first hydrocarbon stream and at least a portion of the products of the dehydrogenation and isomerization reactions form a second hydrocarbon stream, the second hydrocarbon stream comprising olefins and paraffins, and wherein at least a portion of the olefins in the second hydrocarbon stream are branched olefins; and;
introducing at least a portion of the second hydrocarbon stream into a hydroformylation unit, wherein the hydroformylation unit is configured to hydroformylate at least a portion of the olefins in the second hydrocarbon stream to produce aliphatic alcohols, and wherein at least a portion of the produced aliphatic alcohols comprise a branched alkyl group.

2. The method of claim 1, wherein the first hydrocarbon stream is produced from an olefin oligomerization process.

3. The method of claim 1, wherein the first hydrocarbon stream is produced from a Fischer-Tropsch process.

4. The method of any one of claims 1 to 3, wherein the first hydrocarbon stream comprises olefins and paraffins having a carbon number of 7 to 18, particularly a carbon number of 10 to 17.

5. The method of any one of claims 1 to 4, wherein the first hydrocarbon stream comprises an olefin content between 10 percent and 50 percent of the total amount of hydrocarbons in the first hydrocarbon stream.

6. The method of any one of claims 1 to 4, wherein the first hydrocarbon stream comprises 90 percent paraffins.

7. The method of any one of claims 1 to 6, wherein the dehydrogenation-isomerization unit is operated at a temperature range from 300 °C to 500 °C.

8. The method of any one of claims 1 to 7, wherein the dehydrogenation-isomerization unit is configured to operate at a pressure range from 0.10 atmosphere to 15 atmospheres.

9. The method of any one of claims 1 to 8, wherein a residence time of at least a portion of the first hydrocarbons stream in the dehydrogenation-isomerization unit is such that the conversion level of the paraffins in the first hydrocarbon stream to olefins is less than 50 mole percent.

10. The method of any one of claims 1 to 9, further comprising:
introducing at least a portion of the second hydrocarbon stream into a separation unit, wherein the separation unit is configured to separated at least a portion of the branched olefins from linear olefins and paraffins to from a linear olefins and paraffins stream and a branched olefins stream;
combining at least a portion of the linear olefins and paraffins stream with the first hydrocarbon stream upstream of the dehydrogenation-isomerization unit; and,
combining at least a portion of the branched olefins stream with the second hydrocarbon stream upstream of the hydroformylation unit.

11. The method of any one of claims 1 to 10, wherein the aliphatic alcohols produced by the hydroformylation unit comprise greater than 50 percent of the total hydrocarbon content of the hydroformylation reaction stream.

12. The method of any one of claims 1 to 11, wherein the hydroformylation unit is operated at a reaction temperature from 100 °C to 300 °C.

13. The method of any one of claims 1 to 12, further comprising adjusting a ratio of olefins to paraffins introduced into the hydroformylation unit by adding at least a portion of a third hydrocarbon stream into the hydroformylation unit.

14. The method of any one of claims 1 to 12, further comprising adjusting a ratio of olefins to paraffins introduced into the hydroformylation unit by adding at least a portion of a third hydrocarbon stream into the hydroformylation unit, wherein the third hydrocarbon stream comprises greater than 80 percent olefins by weight.

15. The method of any one of claims 1 to 12, further comprising adjusting a ratio of olefins to paraffins introduced into the hydroformylation unit by combining at least a portion of a third hydrocarbon stream with at least a portion of the second hydrocarbon stream upstream of the hydroformylation unit and introducing the combined stream into the hydroformylation unit

16. The method of any one of claims 1 to 12, further comprising: adjusting a ratio of olefins to paraffins introduced into the hydroformylation unit by combining at least a portion of a third hydrocarbon stream with at least a portion of the second hydrocarbon stream upstream of the hydroformylation unit, wherein the third hydrocarbon stream comprises greater than 80 percent olefins by weight; and introducing the mixed stream into the hydroformylation unit.

17. The method of any one of claims 1 to 16, further comprising:
separating aliphatic alcohols from the hydroformylation reaction stream to produce at least a paraffins and unreacted olefins stream and an aliphatic alcohols product stream; and,
introducing at least a portion of the paraffins and unreacted olefins stream into the dehydrogenation-isomerization unit.

18. The method of claim 17, wherein introducing at least a portion of the paraffins and unreacted olefins stream into the dehydrogenation-isomerization unit comprises: combining at least a portion of the paraffins and unreacted olefins stream with at least a portion of the first hydrocarbon stream to produce a combined stream upstream of the dehydrogenation-isomerization unit; and introducing at least a portion of the combined stream into the dehydrogenation-isomerization unit.

19. The method of any one of claims 1 to 18, wherein the dehydrogenation-isomerization unit comprises a dehydrogenation-isomerization catalyst configured to catalyze both dehydrogenation reactions and isomerization reactions in the dehydrogenation-isomerization unit.

20. The method of claim 19, the catalyst comprising a hydrogen form of a zeolite having a ferrierite isotypic framework structure, a binder, a coke-oxidizing compound, and a paraffin dehydrogenation-promoting compound.

21. The method of any one of claims 1 to 20, wherein the dehydrogenation-isomerization unit comprises a plurality of zones, wherein the plurality of zones comprises a first reaction zone and a second reaction zone, wherein the first reaction zone is configured to dehydrogenate at least a portion of paraffins to olefins, and wherein the second reaction zone is configured to isomerize at least a portion of linear olefins to branched olefins, and wherein at least a portion of the unreacted components of the first hydrocarbon stream and at least a portion of the products of the dehydrogenation and isomerization reactions form a second hydrocarbon stream.

22. The method of claim 21, wherein the first reaction zone is operated at a temperature of between 300 °C and 600 °C, particularly at a temperature of between 450 °C and 550 °C.

23. The method of claim 21, wherein the first reaction zone is operated at a total reaction pressure between 0.01 atmospheres and 25.0 atmospheres.

24. The method of any one of claims 21 to 23, wherein a residence time of at least a portion of the first hydrocarbon stream in the first reaction zone is such that the conversion level of the paraffins to olefins is less than 50 mole percent.

25. The method of any one of claims 21 to 24, further comprising introducing at least a portion of the first hydrocarbon stream exiting the first reaction zone into a heat exchanger, wherein the heat exchanger is configured to remove heat from a portion of the first hydrocarbon stream before it enters the second reaction zone.

26. The method of any one of claims 21 to 25, wherein the second reaction zone is operated at a temperature range from 200 °C to 500 °C*.*

27. The method of any one of claims 21 to 26, wherein the second reaction zone is operated at a hydrocarbon partial pressure of from 0.1 atmosphere to 10 atmospheres.

28. The method of any one of claims 1 to 27, wherein the dehydrogenation-isomerization unit comprises a stacked bed catalyst configuration, wherein the stacked bed comprises a dehydrogenation catalyst and an isomerization catalyst.

29. The method of any one of claims 1 to 28, further comprising introducing hydrogen into the first hydrocarbon stream.

30. The method of any one of claims 1 to 29, further comprising:
forming a hydroformylation reaction stream, wherein the hydroformylation stream comprises at least a portion of the unreacted hydrocarbons from the second hydrocarbon stream and at a portion of the produced aliphatic alcohols; and,
separating at least a portion of the hydroformylation reaction stream into an aliphatic alcohol product stream and a paraffins and unreacted olefins stream.

31. The method of any one of claims 1 to 29, further comprising: introducing at least a portion of the produced aliphatic alcohols into a sulfation unit, wherein the sulfation unit is configured to sulfate at least a portion of the aliphatic alcohols to produce aliphatic sulfates, and wherein at least a portion of the aliphatic sulfates produced comprise branched aliphatic sulfates.

32. The method of any one of claims 1 to 29, further comprising introducing at least a portion of the produced aliphatic alcohols into an oxyalkylation unit, wherein the oxyalkylation unit is configured to oxyalkylate at least a portion of the aliphatic alcohols to produce oxyalkyl alcohols, wherein at least a portion of the oxyalkyl alcohols produced comprises branched oxyalkyl alcohols.

## Patentansprüche

1. Verfahren zur Herstellung von aliphatischen Alkoholen, umfassend:
das Einführen eines ersten Kohlenwasserstoffstroms, umfassend Olefine und Paraffine, in eine Dehydrierungs-Isomerisierungseinheit, wobei die Dehydrierungs-Isomerisierungseinheit konfiguriert ist, um wenigstens einen Teil der Paraffine in dem ersten Kohlenwasserstoffstrom zu Olefinen zu dehydrieren, und wobei die Dehydrierungs-Isomerisierungseinheit ferner konfiguriert ist, wenigstens einen Teil der linearen Olefine zu verzweigten Olefinen zu isomerisieren, und wobei wenigstens ein Teil der nicht umgesetzten Komponenten des ersten Kohlenwasserstoffstroms und wenigstens ein Teil der Produkte der Dehydrierungs- und Isomerisierungsreaktionen einen zweiten Kohlenwasserstoffstrom ausbilden, welcher zweite Kohlenwasserstoffstrom Olefine und Paraffine umfasst, und wobei wenigstens ein Teil der Olefine in dem zweiten Kohlenwasserstoffstrom verzweigte Olefine sind; und
das Einführen von wenigstens einem Teil des zweiten Kohlenwasserstoffstroms in eine Hydroformylierungseinheit, wobei die Hydroformylierungseinheit konfiguriert ist, um wenigstens einen Teil der olefine in dem zweiten Kohlenwasserstoffstrom zu hydroformylieren, um aliphatische Alkohole herzustellen, und wobei wenigstens ein Teil der hergestellten aliphatischen Alkohole eine verzweigte Alkylgruppe umfasst.

2. Verfahren nach Anspruch 1, wobei der erste Kohlenwasserstoffstrom aus einem Olefinoligomerisierungsverfahren stammt.

3. Verfahren nach Anspruch 1, wobei der erste Kohlenwasserstoffstrom aus einem Fischer-Tropsch-Verfahren stammt.

4. Verfahren nach einem der Ansprüche 1 bis 3, wobei der erste Kohlenwasserstoffstrom Olefine und Paraffine umfasst, die eine Kohlenstoffzahl von 7 bis 18, insbesondere eine Kohlenstoffzahl von 10 bis 17 besitzen.

5. Verfahren nach einem der Ansprüche 1 bis 4, wobei der erste Kohlenwasserstoffstrom einen Olefingehalt von 10 Prozent bis 50 Prozent der gesamten Menge an Kohlenwasserstoffen im ersten Kohlenwasserstoffstrom umfasst.

6. Verfahren nach einem der Ansprüche 1 bis 4, wobei der erste Kohlenwasserstoffstrom 90 Prozent Paraffine umfasst.

7. Verfahren nach einem der Ansprüche 1 bis 6, wobei die Dehydrierungs-Isomerisierungseinheit in einem Temperaturbereich von 300°C bis 500°C betrieben wird.

8. Verfahren nach einem der Ansprüche 1 bis 7, wobei die Dehydrierungs-Isomerisierungseinheit konfiguriert ist, um in einem Druckbereich von 0,10 Atmosphären bis 15 Atmosphären betrieben zu werden.

9. Verfahren nach einem der Ansprüche 1 bis 8, wobei eine Verweilzeit von wenigstens einem Teil des ersten Kohlenwasserstoffstroms in der Dehydrierungs-Isomerisierungseinheit derart ist, dass der Umsetzungsgrad der Paraffine zu Olefinen in dem ersten Kohlenwasserstoffstrom kleiner als 50 Mol-% ist.

10. Verfahren nach einem der Ansprüche 1 bis 9, ferner umfassend:
das Einführen von wenigstens einem Teil des zweiten Kohlenwasserstoffstroms in eine Trenneinheit, wobei die Trenneinheit konfiguriert ist, um wenigstens einen Teil der verzweigten Olefine von den linearen Olefinen und Paraffinen abzutrennen, um einen Strom aus linearen Olefinen und Paraffinen und einen Strom aus verzweigten Olefinen auszubilden;
das Kombinieren von wenigstens einem Teil des Stroms aus linearen Olefinen und Paraffinen mit dem ersten Kohlenwasserstoffstrom, stromaufwärts der Dehydrierungs-Isomerisierungseinheit; und
das Kombinieren von wenigstens einem Teil des Stroms aus verzweigten Olefinen mit dem zweiten Kohlenwasserstoffstrom, stromaufwärts der Hydroformylierungseinheit.

11. Verfahren nach einem der Ansprüche 1 bis 10, wobei die mit der Hydroformylierungseinheit hergestellten Alkohole, mehr als 50 Prozent des gesamten Kohlenwasserstoffgehalts des Hydroformylierungsreaktionsstroms umfassen.

12. Verfahren nach einem der Ansprüche 1 bis 11, wobei die Hydroformylierungseinheit bei einer Reaktionstemperatur von 100°C bis 300°C betrieben wird.

13. Verfahren nach einem der Ansprüche 1 bis 12, ferner umfassend das Einstellen eines Verhältnisses von Olefinen zu Paraffinen, die in die Hydroformylierungseinheit eingeführt werden, durch Zugeben von wenigstens einem Teil eines dritten Kohlenwasserstoffstroms in die Hydroformylierungseinheit.

14. Verfahren nach einem der Ansprüche 1 bis 12, ferner umfassend das Einstellen eines Verhältnisses von Olefinen zu Paraffinen, die in die Hydroformylierungseinheit eingeführt werden, durch Zugeben von wenigstens einem Teil eines dritten Kohlenwasserstoffstroms in die Hydroformylierungseinheit, wobei der dritte Kohlenwasserstoffstrom mehr als 80 Gew.-% Olefine umfasst.

15. Verfahren nach einem der Ansprüche 1 bis 12, ferner umfassend das Einstellen eines Verhältnisses von Olefinen zu Paraffinen, die in die Hydroformylierungseinheit eingeführt werden, durch Kombinieren von wenigstens einem Teil von einem dritten Kohlenwasserstoffstrom mit wenigstens einem Teil des zweiten Kohlenwasserstoffstroms, stromaufwärts zur Hydroformylierungseinheit; und das Einführen des kombinierten Stroms in die Hydroformylierungseinheit.

16. Verfahren nach einem der Ansprüche 1 bis 12, ferner umfassend das Einstellen eines Verhältnisses von Olefinen zu Paraffinen, die in die Hydroformylierungseinheit eingeführt werden, durch Kombinieren von wenigstens einem Teil von einem dritten Kohlenwasserstoffstrom mit wenigstens einem Teil des zweiten Kohlenwasserstoffstroms, stromaufwärts zur Hydroformylierungseinheit, wobei der dritte Kohlenwasserstoffstrom mehr als 80 Gew.-% Olefine umfasst; und das Einführen des gemischten Stroms in die Hydroformylierungseinheit.

17. Verfahren nach einem der Ansprüche 1 bis 16, ferner umfassend:
das Abtrennen von aliphatischen Alkoholen aus dem Hydroformylierungsreaktionsstrom, um wenigstens einen Strom aus Paraffinen und nicht umgesetzten Olefinen und einen Produktstrom aus aliphatischen Alkoholen herzustellen; und
das Einführen von wenigstens einem Teil des Stroms aus Paraffinen und nicht umgesetzten Olefinen in die Dehydrierungs-Isomerisierungseinheit.

18. Verfahren nach Anspruch 17, wobei das Einführen von wenigstens einem Teil des Stroms aus Paraffinen und nicht umgesetzten Olefinen in die Dehydrierungs-Isomerisierungseinheit: das Kombinieren von wenigstens einem Teil des Stroms aus Paraffinen und nicht umgesetzten Olefinen mit wenigstens einem Teil des ersten Kohlenwasserstoffstroms, um einen kombinierten Strom, stromaufwärts zur Dehydrierungs-Isomerisierungseinheit herzustellen; und das Einführen von wenigstens einem Teil des kombinierten Stroms in die Dehydrierungs-Isomerisierungseinheit, umfasst.

19. Verfahren nach einem der Ansprüche 1 bis 18, wobei die Dehydrierungs-Isomerisierungseinheit einen Dehydrierungs-Isomerisierungskatalysator umfasst, welcher konfiguriert ist sowohl die Dehydrierungsreaktionen als auch die Isomerisierungsreaktionen in der Dehydrierungs-Isomerisierungseinheit zu katalysieren.

20. Verfahren nach Anspruch 19, wobei der Katalysator eine Wasserstoffform von einem Zeolith, welcher eine isotypische Ferrierit-Gitternetzwerkstruktur besitzt, ein Bindemittel, eine koksoxidierende Verbindung und eine die Paraffindehydrierung begünstigende Verbindung umfasst.

21. Verfahren nach einem der Ansprüche 1 bis 20, wobei die Dehydrierungs-Isomerisierungseinheit eine vielzahl an Zonen umfasst, wobei die Vielzahl an Zonen eine erste Reaktionszone und eine zweite Reaktionszone umfasst, wobei die erste Reaktionszone konfiguriert ist, um wenigstens einen Teil der Paraffine zu Olefinen zu dehydrieren, und wobei die zweite Reaktionszone konfiguriert ist, um wenigstens einen Teil der linearen Olefine zu verzweigten Olefinen zu isomerisieren, und wobei wenigstens ein Teil der nicht umgesetzten Komponenten des ersten Kohlenwasserstoffstroms und wenigstens ein Teil der Produkte der Dehydrierungs- und Isomerisierungsreaktionen einen zweiten Kohlenwasserstoffstrom ausbilden.

22. Verfahren nach Anspruch 21, wobei die erste Reaktionszone bei einer Temperatur von 300°C bis 600°C, insbesondere bei einer Temperatur von 450°C bis 550°C betrieben wird.

23. Verfahren nach Anspruch 21, wobei die erste Reaktionszone bei einem Gesamtreaktionsdruck von 0,01 Atmosphären bis 25,0 Atmosphären betrieben wird.

24. Verfahren nach einem der Ansprüche 21 bis 23, wobei eine verweilzeit von wenigstens einem Teil des ersten Kohlenwasserstoffstroms in der ersten Reaktionszone derart ist, dass der Umsetzungsgrad der Paraffine zu Olefinen geringer als 50 Mol-% ist.

25. Verfahren nach einem der Ansprüche 21 bis 24, ferner umfassend das Einführen von wenigstens einem Teil des ersten Kohlenwasserstoffstroms, welcher die erste Reaktionszone verlässt, in einen Wärmetauscher, wobei der Wärmetauscher konfiguriert ist, um die Wärme aus einem Teil des ersten Kohlenwasserstoffstroms zu entfernen, bevor er in die zweite Reaktionszone eintritt.

26. Verfahren nach einem der Ansprüche 21 bis 25, wobei die zweite Reaktionszone in einem Temperaturbereich von 200°C bis 500°C betrieben wird.

27. Verfahren nach einem der Ansprüche 21 bis 26, wobei die zweite Reaktionszone bei einem Kohlenwasserstoffpartialdruck von 0,1 Atmosphären bis 10 Atmosphären betrieben wird.

28. Verfahren nach einem der Ansprüche 1 bis 27, wobei die Dehydrierungs-Isomerisierungseinheit eine Schichtbett-Katalysatoranordnung umfasst, wobei das Schichtbett einen Dehydrierungskatalysator und einen Tsomerisierungskatalysator umfasst.

29. Verfahren nach einem der Ansprüche 1 bis 28, ferner umfassen das Einführen von Wasserstoff in den ersten Kohlenwasserstoffstrom.

30. Verfahren nach einem der Ansprüche 1 bis 29, ferner umfassend:
das Ausbilden eines Hydroformylierungsreaktionsstroms, wobei der Hydroformylierungsreaktionsstrom wenigstens einen Teil der nicht umgesetzten Kohlenwasserstoffe aus dem zweiten Kohlenwasserstoffstrom und einen Teil der hergestellten aliphatischen Alkohole umfasst; und
das Abtrennen von wenigstens einem Teil des Hydroformylierungsreaktionsstroms in einem Produktstrom aus aliphatischen Alkoholen und einem Strom aus Paraffinen und nicht umgesetzten Olefinen.

31. Verfahren nach einem der Ansprüche 1 bis 29, ferner umfassend das Einführen von wenigstens einem Teil der hergestellten aliphatischen Alkohole in eine Sulfatierungseinheit, wobei die Sulfatierungseinheit konfiguriert ist, um wenigstens einen Teil der aliphatischen Alkohole zu sulfatieren, um aliphatische Sulfate herzustellen, und wobei wenigstens ein Teil der hergestellten aliphatischen Sulfate verzweigte aliphatische Sulfate umfasst.

32. Verfahren nach einem der Ansprüche 1 bis 29, ferner umfassend das Einführen von wenigstens einem Teil der hergestellten aliphatischen Alkohole in eine Oxyalkylierungseinheit, wobei die Oxyalkylierungseinheit konfiguriert ist, um wenigstens einen Teil der aliphatischen Alkohole zu oxyalkylieren, um oxyalkylierte Alkohole herzustellen, wobei wenigstens ein Teil der hergestellten oxyalkylierten Alkohole verzweigte oxyalkylierte Alkohole umfasst.

## Revendications

1. Procédé pour produire des alcools aliphatiques, comprenant les étapes suivantes :
l'introduction d'un premier courant d'hydrocarbures comprenant des oléfines et des paraffines dans une unité de déshydrogénation-isomérisation, où l'unité de déshydrogénation-isomérisation est configurée pour déshydrogéner au moins une partie des paraffines dans le premier courant d'hydrocarbures sous la forme d'oléfines, et où l'unité de déshydrogénation-isomérisation est également configurée pour isomériser au moins une partie des oléfines linéaires en oléfines ramifiées, et où au moins une partie des composants du premier courant d'hydrocarbures, n'ayant pas réagie, et au moins une partie des produits de la réaction de déshydrogénation-isomérisation, forment un deuxième courant d'hydrocarbures, le deuxième courant d'hydrocarbures comprenant des oléfines et des paraffines, et où au moins une partie des oléfines dans le deuxième courant d'hydrocarbures est constituée d'oléfines ramifiées; et
l'introduction d'au moins une partie du deuxième courant d'hydrocarbures dans une unité d'hydroformylation, où l'unité d'hydroformylation est configurée pour hydroformyler au moins une partie des oléfines dans le deuxième courant d'hydrocarbures pour produire des alcools aliphatiques, et où au moins une partie des alcools aliphatiques comprend un groupement alkyle ramifié.

2. Procédé selon la revendication 1, dans lequel le premier courant d'hydrocarbures est produit à partir d'un procédé d'oligomérisation d'oléfines.

3. Procédé selon la revendication 1, dans lequel le premier courant d'hydrocarbures est produit à partir d'un procédé de Fischer-Tropsch.

4. Procédé selon l'une quelconque des revendications 1 à 3, dans lequel le premier courant d'hydrocarbures comprend des oléfines et des paraffines ayant un nombre d'atomes de carbone compris entre 7 et 18, en particulier, un nombre d'atomes de carbone compris entre 10 et 17.

5. Procédé selon l'une quelconque des revendications 1 à 4, dans lequel le premier courant d'hydrocarbures comprend une teneur en oléfines comprise entre 10 pour-cent et 50 pour-cent de la quantité totale des hydrocarbures dans le premier courant d'hydrocarbures.

6. Procédé selon l'une quelconque des revendications 1 à 4, dans lequel le premier courant d'hydrocarbures comprend 90 pour-cent de paraffines.

7. Procédé selon l'une quelconque des revendications 1 à 6, dans lequel l'unité de déshydrogénation-isomérisation est exploitée dans une plage de températures de 300 °C à 500 °C.

8. Procédé selon l'une quelconque des revendications 1 à 7, dans lequel l'unité de déshydrogénation-isomérisation est configurée pour être exploitée dans une plage de pressions de 0,10 atmosphère à 15 atmosphères.

9. Procédé selon l'une quelconque des revendications 1 à 8, dans lequel un temps de séjour d'au moins une partie du premier courant d'hydrocarbures dans l'unité de déshydrogénation-isomérisation est tel que le niveau de conversion des paraffines dans le premier courant d'hydrocarbures sous forme d'oléfines est inférieure à 50 % en mole.

10. Procédé selon l'une quelconque des revendications 1 à 9, comprenant en outre les étapes suivantes :
l'introduction d'au moins une partie du deuxième courant d'hydrocarbures dans une unité de séparation, où l'unité de séparation est configurée pour séparer au moins une partie des oléfines ramifiées des oléfines linéaires et des paraffines, pour former un courant d'oléfines linéaires et de paraffines et un courant d'oléfines ramifiées.
la combinaison d'au moins une partie du courant d'oléfines linéaires et de paraffines avec le premier courant d'hydrocarbures en amont de l'unité de déshydrogénation-isomérisation; et
la combinaison d'au moins une partie du courant d'oléfines ramifiées avec le deuxième courant d'hydrocarbures en amont de l'unité d'hydroformylation.

11. Procédé selon l'une quelconque des revendications 1 à 10, dans lequel les alcools aliphatiques produits par l'unité d'hydroformylation constituent plus de 50 pour-cent de la teneur totale en hydrocarbures du courant issu de la réaction d'hydroformylation.

12. Procédé selon l'une quelconque des revendications 1 à 11, dans lequel l'unité d'hydroformylation est exploitée à une température réactionnelle de 100 °C à 300 °C.

13. Procédé selon l'une quelconque des revendications 1 à 12, comprenant également le réglage d'un rapport des oléfines aux paraffines, introduites dans l'unité d'hydroformylation, en ajoutant au moins une partie d'un troisième courant d'hydrocarbures dans l'unité d'hydroformylation.

14. Procédé selon l'une quelconque des revendications 1 à 12, comprenant encore le réglage d'un rapport des oléfines aux paraffines introduites dans l'unité d'hydroformylation, en ajoutant au moins une partie d'un troisième courant d'hydrocarbures dans l'unité d'hydroformylation, le troisième courant d'hydrocarbures comprenant plus de 80 pour-cent en poids d'oléfines.

15. Procédé selon l'une quelconque des revendications 1 à 12, comprenant en outre le réglage d'un rapport des oléfines aux paraffines introduites dans l'unité d'hydroformylation, en combinant au moins une partie d'un troisième courant d'hydrocarbures avec au moins une partie du deuxième courant d'hydrocarbures en amont de l'unité d'hydroformylation et en introduisant le courant combiné dans l'unité d'hydroformylation.

16. Procédé selon l'une quelconque des revendications 1 à 12, comprenant également le réglage d'un rapport des oléfines aux paraffines introduites dans l'unité d'hydroformylation, en combinant au moins une partie d'un troisième courant d'hydrocarbures avec au moins une partie du deuxième courant d'hydrocarbures en amont de l'unité d'hydroformylation, le troisième courant d'hydrocarbures comprenant plus de 80 pour-cent en poids d'oléfines; et en introduisant le courant mélangé dans l'unité d'hydroformylation.

17. Procédé selon l'une quelconque des revendications 1 à 16, comprenant en outre :
la séparation des alcools aliphatiques du courant issu de la réaction d'hydroformylation pour produire au moins un courant de paraffines et d'oléfines n'ayant pas réagi, et un courant de produits d'alcool aliphatique;
et,
l'introduction d'au moins une partie du courant de paraffines et d'oléfines n'ayant pas réagi dans l'unité de déshydrogénation-isomérisation.

18. Procédé selon la revendication 17, dans lequel l'introduction d'au moins une partie du courant des paraffines et des oléfines n'ayant pas réagi dans l'unité de déshydrogénation-isomérisation comprend : la combinaison d'au moins une partie du courant des paraffines et des oléfines n'ayant pas réagi avec au moins une partie du premier courant d'hydrocarbures pour obtenir un courant combiné en amont de l'unité de déshydrogénation-isomérisation ; et l'introduction d'au moins une partie du courant combiné dans l'unité de déshydrogénation-isomérisation.

19. Procédé selon l'une quelconque des revendications 1 à 18, dans lequel l'unité de déshydrogénation-isomérisation comprend un catalyseur de déshydrogénation-isomérisation configuré pour catalyser à la fois les réactions de déshydrogénation et les réactions d'isomérisation dans l'unité de déshydrogénation-isomérisation.

20. procédé selon la revendication 19, dans lequel le catalyseur comprend une forme hydrogénée de zéolite, présentant une structure architecturale de type ferriérite, un liant, un composé d'oxydation du coke et un composé favorisant la déshydrogénation des paraffines.

21. Procédé selon l'une quelconque des revendications 1 à 20, dans lequel l'unité de déshydrogénation-isomérisation comprend une pluralité de zones, où la pluralité de zones comprend une première zone réactionnelle et une seconde zone réactionnelle, la première zone réactionnelle étant configurée pour déshydrogéner au moins une partie des paraffines en oléfines, et la seconde zone réactionnelle étant configurée pour isomériser au moins une partie des oléfines linéaires en oléfines ramifiées, et où au moins une partie des composés n'ayant pas réagi du premier courant d'hydrocarbures et au moins une partie des produits des réactions de déshydrogénation et d'isomérisation forment un deuxième courant d'hydrocarbures.

22. Procédé selon la revendication 21, dans lequel la première zone réactionnelle est exploitée à une température comprise dans la plage de 300° C à 600 °C, en particulier, à une température située entre 450° C et 550 °C.

23. Procédé selon la revendication 21, dans lequel la première zone réactionnelle est exploitée à une pression réactionnelle totale comprise dans la plage de 0,01 atmosphère à 25 atmosphères.

24. Procédé selon l'une quelconque des revendications 21 à 23, dans lequel le temps de séjour d'au moins une partie du premier courant d'hydrocarbures dans la première zone réactionnelle est tel que le niveau de conversion des paraffines en oléfines est inférieur à 50 % en mole.

25. Procédé selon l'une quelconque des revendications 21 à 24, comprenant en outre l'introduction d'au moins une partie du premier courant d'hydrocarbures sortant de la première zone réactionnelle dans un échangeur thermique, l'échangeur thermique étant configuré pour dissiper la chaleur d'une partie du premier courant d'hydrocarbures avant qu'il ne pénètre dans la seconde zone réactionnelle.

26. Procédé selon l'une quelconque des revendications 21 à 25, dans lequel la seconde zone réactionnelle est exploitée dans une plage de températures de 200° C à 500° C.

27. Procédé selon l'une quelconque des revendications 21 à 26, dans lequel la seconde zone réactionnelle est exploitée à une pression partielle d'hydrocarbures située dans la plage de 0,1 atmosphère à 10 atmosphères.

28. Procédé selon l'une quelconque des revendications 1 à 27, dans lequel l'unité de déshydrogénation-isomérisation comprend une configuration de catalyseur en lit empilé, le lit empilé comprenant un catalyseur de déshydrogénation et un catalyseur d'isomérisation.

29. Procédé selon l'une quelconque des revendications 1 à 28, comprenant en outre l'introduction d'hydrogène dans le premier courant d'hydrocarbures.

30. Procédé selon l'une quelconque des revendications 1 à 29, comprenant également :
la formation d'un courant de réaction d'hydroformylation, le courant d'hydroformylation comprenant au moins une partie des hydrocarbures n'ayant pas réagi du deuxième courant d'hydrocarbures et une partie des alcools aliphatiques produits; et
la séparation d'au moins une partie du courant de réaction d'hydroformylation sous la forme d'un courant de produits d'alcools aliphatiques et d'un courant de paraffines et d'oléfines n'ayant pas réagi.

31. Procédé selon l'une quelconque des revendications 1 à 29, comprenant en outre l'introduction d'au moins une partie des alcools aliphatiques produits dans une unité de sulfatation, l'unité de sulfatation étant configurée pour sulfater au moins une partie des alcools aliphatiques pour produire des sulfates aliphatiques, et au moins une partie des sulfates aliphatiques produits comprenant des sulfates aliphatiques ramifiés.

32. Procédé selon l'une quelconque des revendications 1 à 29, comprenant en outre l'introduction d'au moins une partie des alcools aliphatiques produits dans une unité d'oxyalkylation, l'unité d'oxyalkyation étant configurée pour effectuer l'oxyalkylation d'au moins une partie des alcools aliphatiques pour produire des oxyalkylalcools ; où au moins une partie des oxyalkylalcools produits comprend des oxyalkylalcools ramifiés.
